(19) Europäisches Patentamt
European Patent Office
Office européen des brevets

(11) **EP 0 799 890 B1**

(12) **EUROPÄISCHE PATENTSCHRIFT**

(45) Veröffentlichungstag und Bekanntmachung des Hinweises auf die Patenterteilung:
**27.05.2009 Patentblatt 2009/22**

(51) Int Cl.:
*C12N 15/12* (2006.01)    *C12N 15/31* (2006.01)
*C07K 14/36* (2006.01)    *C07K 14/465* (2006.01)
*G01N 33/53* (2006.01)

(21) Anmeldenummer: **97105408.5**

(22) Anmeldetag: **01.04.1997**

(54) **Rekombinante inaktive Core-Streptavidin Mutanten**

Recombinant inactive core streptavidin mutants

Mutants inactifs de la partie centrale de la streptavidine

(84) Benannte Vertragsstaaten:
**DE ES FR IT**

(30) Priorität: **01.04.1996 DE 19613053**
**16.09.1996 DE 19637718**

(43) Veröffentlichungstag der Anmeldung:
**08.10.1997 Patentblatt 1997/41**

(73) Patentinhaber: **Roche Diagnostics GmbH**
**68305 Mannheim (DE)**

(72) Erfinder:
• **Kopetzki, Eberhard, Dr.**
**82377 Penzberg (DE)**
• **Müller, Rainer, Dr.**
**82377 Penzberg (DE)**
• **Engh, Richard, Dr.**
**81375 München (DE)**
• **Schmitt, Urban**
**82386 Oberhausen (DE)**
• **Deger, Arno, Dr.**
**82377 Penzberg (DE)**
• **Brandstetter, Hans, Dr. rer. nat.**
**Chelsea, MA 02150 (US)**

(74) Vertreter: **Weiss, Wolfgang et al**
**Weickmann & Weickmann**
**Patentanwälte**
**Kopernikusstrasse 9**
**81679 München (DE)**

(56) Entgegenhaltungen:
**WO-A-96/24606          WO-A-97/00329**

• **TAKESHI SANO ET AL: "Intersubunit contacts made by tryptophan 120 with biotin are essential for both strong biotin binding and biotin-induced tighter subunit association of streptavidin" PROCEEDINGS OF THE NATIONAL ACADEMY OF SCIENCES OF USA, Bd. 92, April 1995 (1995-04), Seiten 3180-3184, XP002108284 WASHINGTON US**
• **GITLIN G ET AL: "Studies on the biotin-binding sites of avidin and streptavidin" THE BIOCHEMICAL JOURNAL, Bd. 269, Nr. 2, Juli 1990 (1990-07), Seiten 527-530, XP002108285 U.K.**

**Beschreibung**

Die vorliegende Erfindung betrifft Muteine von Streptavidin mit einer verringerten Bindungsaffinität für Biotin sowie deren Verwendung als Entstörungsreagenzien in Verfahren zur Bestimmung eines, Analyten, z.B. in diagnostischen Tests wie etwa in Immuno- und Nukleinsäurehybridisierungsassays. Weiterhin betrifft die Erfindung die Verwendung von Muteinen von Streptavidin als regenerierbare Systeme zur Bindung von Biotin, z.B. zur Analyse biotinylierter Moleküle, zur Untersuchung von Rezeptor-Ligand-Wechselwirkungen sowie zur Affinitätsreinigung biotinylierter Moleküle.

In Nachweisverfahren zur Bestimmung von Analyten wie etwa Immuno- und Nukleinsäurehybridisierungsassays erfolgt die Bestimmung des Analyten häufig über eine hochaffine Wechselwirkung zwischen den Partnern eines spezifischen Bindungspaares. Ein typisches Beispiel für ein spezifisches Bindungspaar stellt der Avidin/Streptavidin-Biotin-Komplex dar. Bei der Verwendung des Avidin/Streptavidin-Biotin-Bindungspaares wird dessen hohe Bindungsaffinität genutzt. Dabei wird beispielsweise eine mit Avidin/Streptavidin beschichtete Festphase verwendet, an die ein biotinylierter Komplex aus Analyt und spezifischem Rezeptor binden kann. In anderen Testformaten kann Avidin/Streptavidin auch in löslicher Form eingesetzt werden.

Neben den spezifischen Wechselwirkungen treten jedoch oftmals auch Nebenreaktionen, wie beispielsweise unerwünschte Wechselwirkungen und unspezifische Bindereaktionen zwischen den Testkomponenten und weiteren in der Probe oder an der Festphase vorliegenden Bestandteilen auf. Insbesondere binden an immobilisiertes oder lösliches Avidin und Streptavidin oftmals weitere in der Testprobe vorliegende Substanzen und verursachen dadurch falsch positive oder falsch negative Testergebnisse. Weiterhin können diese Wechselwirkungen auch eine Erhöhung des Hintergrundsignals und eine stärkere Streuung der Signale bewirken, wodurch die Sensitivität und Spezifität des betreffenden Tests herabgesetzt wird.

Es wurden verschiedene Versuche unternommen, diese unspezifischen Wechselwirkungen zu reduzieren. So ist es beispielsweise bekannt, daß unterschiedliche Kohlenhydratkomponenten und unterschiedliche Proteine, Proteingemische oder Proteinfraktionen sowie deren Hydrolysate unspezifische Wechselwirkungen zwischen den Testkomponenten und dem Analyten in Immunoassays reduzieren können (Robertson et al., J. of Immun. Med. 26 (1985) 195; EP-A-260 903; US-A-4,931,385). Der Einsatz von derartigen Kohlenhydrat- und Proteinkomponenten hat jedoch den Nachteil, daß durch darin enthaltene Bestandteile wiederum weitere Störungen des Tests ausgelöst werden können. Enzymatisch hergestellte Hydrolysate können zudem mit den bei der Herstellung verwendeten Proteasen verunreinigt sein und weisen in der Regel keine einheitliche Qualität auf, da sich die Spaltung nur schwer steuern läßt. Solche Verunreinigungen von Proteasen können Testkomponenten angreifen und schon in geringen Mengen zur Beeinträchtigung der Testfunktionen und der Lagerstabilität führen.

Weiterhin wurde zur Verringerung unspezifischer Wechselwirkungen auch der Einsatz von chemisch modifizierten Proteinen, insbesondere von succinylierten oder acetylierten Proteinen beschrieben (US-A-5,051,356; EP-A-0 525 916). Mit diesen Substanzen können jedoch viele der falsch positiven oder falsch negativen Ergebnisse bei Tests auf Antikörper aus Serum nicht vermieden werden.

Zur Vermeidung von unspezifischen Wechselwirkungen wurde weiterhin vorgeschlagen, den Testreagenzien ultrafeine Partikel mit einer maximalen Durchschnittsgröße von 0,2 μm zuzusetzen, welche so ausgebildet sind, daß sie an die Störkomponenten binden und sie abfangen (EP 0163 312). Hierzu ist jedoch eine spezielle Vorbereitung dieser ultrafeinen Partikel nötig, und zudem muß die Art der in der Probe vorhandenen unspezifischen Faktoren bekannt sein.

In DE-A-44 07 423 und DE-A-44 34 093 wurde vorgeschlagen, Störungen, die aufgrund von unspezifischen Wechselwirkungen zwischen Probenbestandteilen und einer Streptavidin-beschichteten Festphase auftreten mittels einer Vorreaktion abzufangen. Die Vorreaktion wird geeigneter Weise an einer Festphase durchgeführt, die der aktiven Streptavidin-beschichteten Festphase möglichst ähnlich ist, an die aber die Probemoleküle nicht spezifisch binden können. Die unspezifischen Bestandteile binden dagegen auch an die inaktive Festphase und können dadurch entfernt werden.

Gemäß DE-A-44 07 423 kann Streptavidin durch kovalente Derivatisierung oder kovalente Modifizierung inaktiviert werden. Nachteilig ist es jedoch, daß dabei eine aufwendige nachträgliche chemisch Modifizierung notwendig ist. Zudem kann durch eine chemische Derivatisierung der Bereich um das aktive Zentrum des nativen Streptavidins auf unerwünschte Weise verändert werden, wodurch sich die Entstörungswirkungen verschlechtern und sogar zusätzliche störende Wechselwirkungen auftreten können. Unspezifische Wechselwirkungen, die an der Biotinbindungstasche auftreten, können durch kovalente Modifizierungen nicht entstört werden.

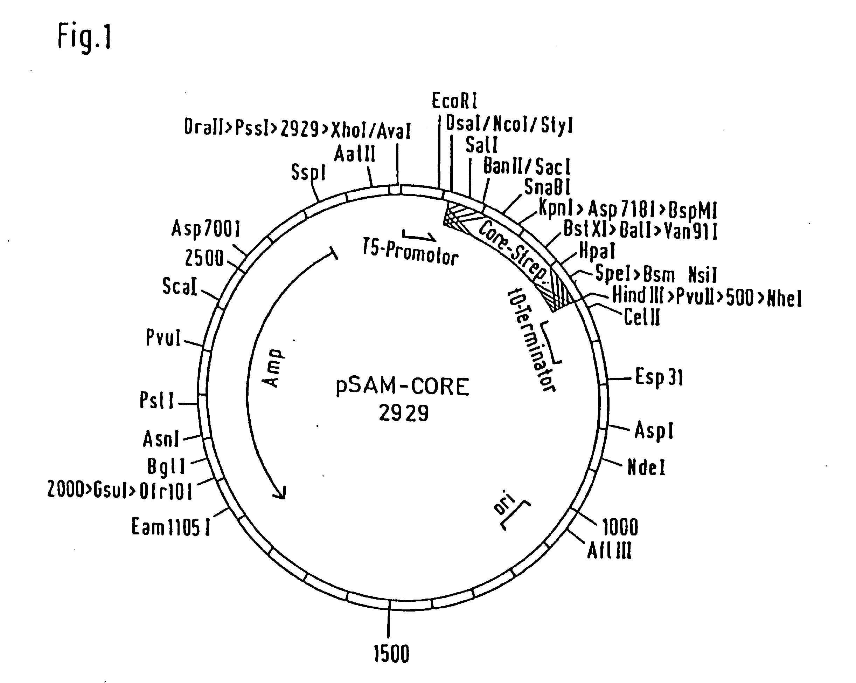

Das Avidin/Streptavidin-Biotin-System ist aufgrund seiner starken, nicht-kovalenten Affinität der Bindungspartner ($K_A$ ungefähr $10^{15}$ 1/mol) Gegenstand zahlreicher Untersuchungen. Die hohe Bindungsaffinität wurde vor allem auf Wechselwirkungen zwischen Tryptophanresten des Streptavidins und Biotin zurückgeführt. Durch Veränderung der Tryptophanreste (Chilkoti et al., Proc. Natl. Acad. Sci. USA 92 (1995) 1754 - 1758, Sano und Cantor, Proc. Natl. Acad. Sci. USA 92 (1995) 3180 - 3184) konnte jedoch eine signifikante Verringerung der Bindungsaffinität der Streptavidinvarianten zu Iminobiotin erzielt werden. Ein eindeutiger Nachweis für eine Verringerung der Hindungsaffinität zu Biotin konnte hingegen nicht gezeigt werden (vgl. Chilkoti et al., Supra, Fig. 1A und B). Für eine Verwendung als Entstörungsreagenz sind solche Varianten deshalb ungeeignet, da sie aufgrund ihrer immer noch recht hohen Bindungsaffinität auch spezifische Reaktionen mit biotinylierten Testkomponenten eingehen können.

Es war daher eine Aufgabe der vorliegenden Erfindung, ein Reagenz bereitzustellen, mittels dessen störende Einflüsse auf Nachweisverfahren zur Bestimmung eines Analyten, z.B. Immuno- oder Nukleinsäurehybridisierungsassays, verringert werden können.

Diese Aufgabe wird erfindungsgemäß gelöst durch ein Biotin-bindefähiges Polypeptid, ausgewählt aus Muteinen von Streptavidin, worin sich das Mutein (a) um mindestens eine Aminosäure vom nativen Polypeptid der SEQ ID No. 2 unterscheidet, wobei mindestens eine der Aminosäuren an den Positionen Leu25, Thr90, Leu110 oder/und Asp128 durch eine andere Aminosäure ausgetauscht ist und (b) eine Bindungsaffinität zu Biotin von weniger als $10^{10}$ l/mol aufweist. Die Bindungsaffinität für die Reaktion

**Streptavidin/Biotin-Komplex ⇌ Streptavidin + Biotin**

beträgt etwa $10^{15}$ 1/mol. Das als Fängersystem verwendete Streptavidin/Biotin-system verfügt damit über eine der stärksten bekannten nicht-kovalenten Wechselwirkungen zwischen einem Protein und einem Liganden. Überraschenderweise wurde festgestellt, daß durch

Austausch von einer oder mehreren Aminosäuren an den Positionen Leu25, Thr90, Leu110 oder/und Asp128 von Streptavidin der SEQ ID No. 2 ein Polypeptid erhalten wird, das einerseits bei rekombinanter Herstellung renaturierbar ist und andererseits eine herabgesetzte Bindungsaffinität zu Biotin auf $< 10^{10}$ 1/mol herabgesetzt werden kann, wobei die erfindungsgemäßen Muteine vorzugsweise weiterhin eine Struktur aufweisen, die der Struktur des aktiven Polypeptids entspricht. Die erfindungsgemäßen Muteine zeigen vorzugsweise eine hohe immunologische Kreuzreaktivität mit dem nativen Polypeptid. Weiterhin ist es bevorzugt, daß sie zur Dimerisierung bzw. Tetramerisierung in der Lage sind. Überraschenderweise besitzen die Muteine trotz der herabgesetzten Bindungsaffinität zu Biotin wie natives Streptavidin die Fähigkeit zur Bindung an störende Probenbestandteile, wie sie in biologischen Proben, z.B. Körperflüssigkeiten wie Serum, Plasma, Vollblut etc. vorkommen können.

Aufgrund ihrer besonderen Eigenschaften können die erfindungsgemäßen Muteine für verschiedenste Anwendungen eingesetzt werden. Ein Herabsetzen der Bindungsaffinität ist gleichbedeutend mit der Verringerung der Wechselwirkungen zwischen Biotin und den Muteinen. Erfindungsgemäße Muteine können so ausgestaltet sein, daß sie überhaupt nicht an Biotin binden, oder aber daß eine relativ lockere reversible Bindung vorliegt. Da die räumliche Struktur der Muteine im Vergleich zum nativen Polypeptid vorzugsweise nicht signifikant verändert ist, werden Wechselwirkungen mit anderen Substanzen nicht beeinflußt. Auf diese Weise wird ein Reagenz erhalten, das in seiner räumlichen Struktur und seinen Bindungseigenschaften dem nativen Streptavidin entspricht, mit Ausnahme der veränderten Bindefähigkeit zu Biotin.

Als Testproben können allgemein wäßrige Proben eingesetzt werden. Insbesondere werden biologische Proben wie Körperflüssigkeiten wie z.B. Vollblut, Blutplasma, Serum, Speichel, Gewebsflüssigkeit, Liquor oder Urin verwendet.

Der Austausch von bestimmten Aminosäuren durch Mutagenese erlaubt eine definierte Herstellung von Muteinen. Im Gegensatz zu anderen Modifizierungen von Streptavidin, wie beispielsweise chemischer Derivatisierung, bleibt die Struktur der erfindungsgemäßen Muteine erhalten. Es können somit keine störenden Wechselwirkungen zwischen zusätzlich eingeführten Derivatisierungsreagenzien und Bestandteilen der zu testenden Probe auftreten.

Die Bindungsaffinität der erfindungsgemäßen Muteine zu Biotin beträgt bevorzugt $< 10^9$ l/mol, stärker bevorzugt $< 10^8$ l/mol, noch stärker bevorzugt $< 10^7$ l/mol, besonders bevorzugt $< 10^6$ l/mol und am meisten bevorzugt $< 10^5$ l/mol.

Bevorzugt zeigen erfindungsgemäße Streptavidin muteine eine Regenerierbarkeit bei Immobilisierung auf einer Sensorchip-Oberfläche, z.B. einer BIAcore-Oberfläche.

Bei dem erfindungsgemäßen Streptavidinmutein werden eine oder mehrere Aminosäuren an den Positionen Leu25, Ser27, Tyr43, Ser45, Val47, Gly48, Ser88, Thr90, Leu110 oder/und Asp128 durch eine andere Aminosäure ausgetauscht. Durch den Austausch einer Aminosäure mit einem kleinen Rest durch eine Aminosäure mit einem größeren Rest kann eine Verringerung der Bindefähigkeit von Biotin erreicht werden, beispielsweise durch den Austausch von Leu oder Ser gegen Trp, Arg, Tyr, Phe oder His. Weiterhin kann die Biotinbindungsfähigkeit auch durch eine zusätzlich eingeführte Disulfidbrücke, die die Zugänglichkeit der Biotinbindungstasche verringert, vermindert oder blockiert werden. Eine zusätzliche Disulfidbrücke kann beispielsweise durch den Austausch von zwei Aminosäuren durch zwei Cysteine im passenden räumlichen Abstand ausgebildet werden. Es ist auch möglich, die Biotinbindungsfähigkeit durch zusätzliche ionische Wechselwirkungen zu verringern. Dazu kann beispielsweise eine positiv geladene Aminosäure wie Arg oder Lys eingeführt werden, die mit Asp128 ionische Wechselwirkungen ausbildet. Andererseits können auch eine positiv geladene und eine negativ geladene Aminosäure eingeführt werden, die miteinander eine Salzbrücke ausbilden und damit die Biotinbindungstasche blockieren können. Vorzugsweise werden kleine und an der Oberfläche lokalisierte Aminosäuren wie z.B. Leu25, Ser27, Ser 45 oder/und Leu110 durch voluminösere Aminosäuren wie z.B. Arg, Trp, Tyr, Phe oder His ausgetauscht.

Zur Erzielung der gewünschten Bindungsaffinität können auch Muteine von Streptavidin gebildet werden, in denen mindestens zwei Aminosäuren ausgetauscht sind. Bevorzugt werden mindestens zwei der Aminosäuren Leu25, Ser27, Ser45 und Leu110, z.B. die Aminosäurepaare Leu25 und Ser45, Ser27 und Ser45 sowie Ser 45 und Leu 110 durch geeignete Aminosäuren, insbesondere voluminösere Aminosäuren wie Arg, Trp, Tyr, Phe oder His ausgetauscht. Besonders bevorzugt werden Leu25 durch Trp und Ser45 durch Arg, Ser27 durch Arg und Ser45 durch Arg, Ser45 durch Trp und Leu110 durch Trp oder Ser45 durch Tyr und Leu110 durch Trp ausgetauscht.

Auch der Austausch von mehr als zwei Aminosäuren führt bei Streptavidin zu erfindungsgemäßen Muteinen. Vorzugsweise werden bei solchen Streptavidin-Mehrfachmutanten mindestens drei der Aminosäuren Leu25, Ser27, Ser45 und Leu110 vorzugsweise durch voluminösere Aminosäuren wie zuvor definiert ausgetauscht. In spezifischen Beispielen für Dreifach-Kombinationsmutanten wurde Leu25 gegen Trp, Ser45 gegen Trp und Leu110 gegen Trp oder Leu25 durch Trp, Ser45 durch Tyr und Leu110 durch Trp ausgetauscht.

In einer weiteren bevorzugten Mehrfach-Kombinantionsmutante sind mindestens zwei der Aminosäuren Leu25, Ser27, Ser45 und Leu110 sowie zusätzlich Trp120 mutagenisiert. Ein spezifisches Beispiel für eine solche Dreifachmutante enthält Austausche von Ser27 gegen Arg, Ser45 gegen Arg und Trp120 gegen Ala.

Die für natives Streptavidin kodierende DNA-Sequenz ist in SEQ ID NO. 1 dargestellt. Die entsprechende Proteinsequenz ist in SEQ ID NO. 2 dargestellt. Die angegebenen Nummern von Aminosäuren beziehen sich auf diese Sequenz. Die Nukleinsäuresequenz von nativem Avidin ist zum Vergleich in SEQ ID NO. 3 dargestellt. SEQ ID NO. 4 zeigt die Aminosäuresequenz von Avidin. Die Nummerierung der Aminosäuren erfolgt entsprechend dieser Sequenz. Muteine von Avidin sind nicht Gegenstand der Erfindung.

Erfindungsgemäß können Muteine auch von Streptavidinvarianten hergestellt werden, die in ihrer ursprünglichen Form mit Biotin bindefähig sind. Erfindungsgemäß bevorzugt sind Muteine eines verkürzten Streptavidins (rekombinantes Core-Streptavidin). Dieses Core-Streptavidin wird vorzugsweise von der in SEQ ID NO. 15 angegebenen Nucleotidsequenz kodiert und enthält in Sequenz ID NO. 16 gezeigte Aminosäuresequenz. Die Herstellung dieses Core-Streptavidins ist in WO 93/09144 beschrieben. Muteine des Core-Streptavidins enthalten Mutationen, wie sie bereits für natives Streptavidin beschrieben wurden.

Ein weiterer Gegenstand der vorliegenden Erfindung ist eine für ein Streptavidin Muteine kodierende Nukleinsäure. Diese Nukleinsäure kann z.B. durch ortsspezifische in vitro Mutagenese einer Nukleinsäure gemäß SEQ ID NO. 1, SEQ ID NO.3 oder SEQ ID NO. 15 hergestellt werden. Die erfindungsgemäße Nukleinsäure kann auf einem Vektor lokalisiert sein, z.B. auf einem prokaryontischen Plasmid, vorzugsweise einem in E.coli replizierbaren Plasmid. Die Nukleinsäure befindet sich auf dem Vektor vorzugsweise in operativer Verknüpfung mit einem Promotor, der eine Expression im jeweiligen Wirtsorganismus erlaubt.

Noch ein weiterer Gegenstand der Erfindung ist eine Zelle, die mit einem Vektor, der ein Streptavidinmutein-Gen enthält, transformiert ist. Vorzugsweise ist die Zelle eine prokaryontische Zelle, insbesondere eine gram-negative Bakterienzelle, z.B. eine E.coli Zelle.

Die Muteine werden vorzugsweise durch rekombinante Expression in einer geeigneten Wirtszelle, insbesondere einer prokaryontischen Wirtszelle wie etwa E. coli hergestellt. Dabei fallen die Muteine üblicherweise in Form von inaktiven inclusion bodies an, die unter geeigneten Bedingungen renaturiert werden können.

Die Muteine können nach der Renaturierung und Reinigung ohne weitere Behandlung in löslicher Form oder nach Immobilisierung auf einer Festphase als Entstörungsreagenz eingesetzt werden. Weiterhin können die Muteine auch in Form eines löslichen oder immobilisierten Polymer-Konjugats eingesetzt werden. Derartige Polymer-Konjugate können durch chemische Kopplung von mehreren Muteinmolekülen untereinander oder durch Kopplung mit anderen Makromolekülen wie etwa Polypeptiden, Proteinen, Kohlenhydraten etc. hergestellt werden.

Bevorzugt sind Konjugate des Muteins mit einem weiteren Polypeptid oder Protein. Besonders bevorzugt handelt es sich um ein Konjugat mit einem Albumin, z.B. Rinderserumalbumin. Die Herstellung von Polymer-Konjugaten der Muteine und gegebenenfalls weiterer Makromoleküle kann nach bekannten Methoden erfolgen (siehe z.B. EP-A-0 269 092).

Weiterhin betrifft die Erfindung die Verwendung eines der oben beschriebenen Muteine als Entstörungsreagenz für Assays zum Nachweis eines Analyten, in denen das Streptavidin -Biotin-Bindepaar als Testkomponente enthalten ist. Dabei können die Muteine in löslicher oder/und immobilisierter Form eingesetzt werden. In einer Ausführungsform können die Muteine zusammen mit einer nicht-modifizierten Streptavidin Festphase in einem heterogenen Assay, z.B. einem immunologischen oder/und Hybridisierungsassay, verwendet werden. Dabei können die Muteine dem Testansatz in löslicher oder/und immobilisierter Form, z.B. in Form einer separaten Festphase, zugesetzt werden.

Der Test kann als Einschritt- oder Zweischrittverfahren durchgeführt werden, d.h. die zu bestimmende Probe kann mit Mutein und nicht-modifiziertem Streptavidin zusammen oder in separaten Reaktionsschritten in Kontakt gebracht werden.

Darüberhinaus können die erfindungsgemäßen Muteine auch in anderen Testformaten, z.B. in homogenen Assays, Agglutinationsassays etc. eingesetzt werden, sofern als Testkomponenten das Streptavidin/- Avidin-Biotin-Bindepaar vorhanden ist. In diesem Zusammenhang umfaßt der Begriff "Biotin" sowohl Biotin in freier Form als auch in Form von biotinylierten Substanzen, wie etwa biotinylierten Nukleinsäuren, Kohlenhydraten, Lipiden, Peptiden oder Polypeptiden. Weiterhin umfaßt der Begriff auch Biotinanaloga und -derivate wie etwa Iminobiotin, Desthiobiotin und Streptavidinaffinitätspeptide.

Ein weiterer Gegenstand der Erfindung ist ein Entstörungsreagenz zur Verringerung oder/und Vermeidung von unspezifischen Wechselwirkungen in einem Verfahren zur Bestimmung eines Analyten, wobei das Entstörungsreagenz eines der oben beschriebenen Muteine enthält. Dieses Entstörungsreagenz kann in löslicher Form vorliegen oder/und an einer Festphase immobilisiert sein, bevorzugt an einer Membran, einer Mikrotiterplatte, einem Mikroreagenzgefäß oder an Mikrobeads. Durch das Entstörungsreagenz können Substanzen, die unspezifische Wechselwirkungen mit Assaybestandteilen eingehen, abgefangen werden, wodurch eine Verbesserung der Testsensitivität erreicht wird. Das Entstörungsreagenz weist im Vergleich zu nicht-modifiziertem Avidin bzw. Streptavidin eine im wesentlichen unveränderte Bindungsfähigkeit gegenüber den Störkomponenten auf, so daß störende Bestandteile der Testprobe wirksam abgefangen werden. Im Gegensatz zu nativem Avidin bzw. Streptavidin weist das Entstörungsreagenz jedoch praktisch eine für den Test zu vernachlässigende Affinität zu Biotin auf, weshalb der Nachweis des Analyten in der Testprobe nicht wesentlich beeinflußt wird.

Ein weiterer Gegenstand der Erfindung ist ein Verfahren zum qualitativen und/oder quantitativen Nachweis eines Analyten in einer Testprobe, umfassend die Verwendung des spezifischen Bindepaars Streptavidin Biotin, wobei man als Entstörungsreagenz ein erfindungsgemäßes Mutein von Streptavidin mit einer Bindungsaffinität zu Biotin < $10^{10}$ l/mol zugibt.

In einer Ausführungsform der vorliegenden Erfindung ist das Verfahren ein heterogener Assay, bei dem die Bestimmung des Analyten über die Bindung an eine Festphase erfolgt, wobei an dieser Festphasenbindung das Streptavidin Bindepaar beteiligt ist.

In einer bevorzugten Ausführungsform dieses Testformats wird eine Festphase verwendet, die mit Streptavidin beschichtet ist und an die eine biotinylierte Testkomponente binden soll. Einem derartigen Testformat kann das erfindungsgemäße Entstörungsreagenz in löslicher oder/und immobilisierter Form zugesetzt werden. Ein immobilisierte Entstörungsreagenz wird vorzugsweise in Form einer separaten inaktiven Festphase zugesetzt, wobei die Testprobe entweder zuerst mit der inaktiven Festphase alleine und erst später mit der aktiven Festphase oder gleichzeitig mit aktiver und inaktiver Festphase in Kontakt gebracht wird.

Bei Verwendung eines löslichen Entstörungsreagenz ist ein Einschritt-Test bevorzugt, bei dem das Entstörungsreagenz zusammen mit allen anderen Testkomponenten in der Probenflüssigkeit vorliegt.

Auch bei Verwendung einer biotinylierten Festphase und löslichem Streptavidin als Testkomponente kann das erfindungsgemäße Entstörungsreagenz erfolgreich eingesetzt werden, z.B. in löslicher Form oder in Form einer separaten inaktiven Festphase.

Der Nachweis des Analyten im erfindungsgemäßen Verfahren kann auf an sich bekannte Weise über eine indirekte oder direkte Markierung erfolgen. Mit direkt markierten Nachweisreagenzien erfolgt der Nachweis dadurch, daß das Nachweisreagenz z.B. an den Analyten bindet und einen detektierbaren Komplex bildet, der die Markierung trägt oder kompetitiv zu dem Analyten an eine spezifische Bindungsstelle bindet. Nachweisreagenzien mit indirekter Markierung umfassen mehrere Komponenten, wobei die Komponente die an den Analyten bindet unmarkiert ist und mit einer weiteren Komponente, die eine Markierung trägt, bindefähig ist.

Geeignete Markierungen sind dem Fachmann bekannt. Es können z.B. radioaktive Markierungen, Chemilumineszenz-, Fluoreszenz- oder Elektrochemilumineszenzmarker, gefärbte Partikel wie z.B. Metallsolpartikel oder gefärbter oder ungefärbter Latex verwendet werden. Die Markierung kann auch ein indirektes Signal liefern wie z.B. bei Enzymmarkierungen mit Enzymen wie Peroxidase, ß-Galactosidase oder alkalischer Phosphatase.

Ein weiterer Gegenstand der Erfindung ist ein Testkit zum qualitativen oder/und quantitativen Nachweis eines Analyten in einer Testprobe, der ein Biotin-bindefähiges Polypeptid sowie weitere Komponenten des jeweiligen Assays und ein Entstörungsreagenz enthält, das ein erfindungsgemäßes Mutein umfaßt. Das Entstörungsreagenz kann in löslicher Form vorliegen oder auf einer Festphase, insbesondere auf einer Mikrotiterplatte, einem Mikroreagenzgefäß, einer Membran oder auf Mikrobeads immobilisiert sein.

Ein weiterer Gegenstand der Erfindung ist die Verwendung von Muteinen von Streptavidin, in denen mindestens eine der Aminosäuren an den Positionen Leu25, Ser27, Typ43, Ser45, Va147, Gly48, Ser88, Thr90, Leu110 oder/und Asp128 des nativen Polypeptids der SEQ ID No. 2 durch eine andere Aminosäure ausgetauscht ist und die eine Bindungsaffinität zu Biotin im Bereich von $10^3$ bis $10^{11}$ l/mol aufweisen, als regenerierbares System zur Bindung biotinylierter Substanzen. Bevorzugt werden Muteine mit einer Bindungsaffinität zu Biotin im Bereich von $10^5$ bis $10^{10}$ l/mol und insbesondere $10^5$ bis $10^6$ l/mol verwendet.

Das regenerierbare System zur Bindung biotinylierter Substanzen umfaßt vorzugsweise eine Festphase, an der die Streptavidinmuteine immobilisiert sind. Beispiele für geeignete Festphasen sind Sensorchips (z.B. das Biacore-System der Fa. Kabi Pharmacia), Reaktionsgefäße wie etwa Polystyrolröhrchen oder Küvetten, Microtiterplatten, Microbeads, Latexpartikel und Trägermaterialien für Affinitätssäulen. Unter der Bezeichnung "biotinylierte Substanzen" sind insbesondere Biotin- und Biotinanaloga-Konjugate zu verstehen, wobei Biotinanaloga solche Substanzen sind, die mit der Biotinbindungstasche von (nativem) Streptavidin einen Komplex ausbilden wie etwa Iminobiotin, Desthiobiotin und Streptavidinaffinitätspeptide.

Der Vorteil der erfindungsgemäßen regenerierbaren Festphasen gegenüber einer mit nativem Streptavidin beschichteten Festphase besteht darin, daß einerseits eine ausreichend hohe Bindungsaffiniät für Biotin (in Form von freiem Biotin oder biotinylierten Substanzen) besteht, so daß die Festphase in Assays zum Nachweis von Analyten, zur Untersuchung von Rezeptor-Ligand-Wechselwirkungen und zur Reinigung bzw. Analyse biotinylierter Substanzen eingesetzt werden kann. Andererseits ist die Bindungsaffinität der Festphase zu Biotin oder einer biotinylierten Substanz ausreichend gering, daß eine Regenerierung der Festphase, d.h. eine Ablösung des Biotins möglich ist. Diese Ablösung erfolgt vorzugsweise durch Verringerung des pH-Werts auf pH < 4,5 oder/und durch Zugabe chaotroper Substanzen, d.h. Substanzen, welche die Ausbildung von Wasserstoffbrückenbindungen stören. Alternativ kann die Ablösung zur Isolierung der biotinylierten Substanzen auch durch Zugabe von freiem Biotin oder/und Biotinanaloga erfolgen. Besonders bevorzugt ist die Gradientenelution.

In einer bevorzugten Ausführungsform wird die regenerierbare Festphase als Affinitätsmatrix eingesetzt. Diese Affinitätsmatrix dient zur Reinigung von biotinylierten Substanzen beispielsweise biotinylierten Antikörpern bzw. zur Auftrennung solcher Substanzen nach der Anzahl der pro Molekül angebundenen Biotinreste. Werden als Matrix nicht-modifiziertes Streptavidin oder Avidin verwendet, können die mit der Matrix in Kontakt gebrachten Substanzen aufgrund der hohen Bindungsaffinität praktisch nicht wieder freigesetzt werden. Im Stand der Technik wurde zur Lösung dieses Problems versucht, Avidin durch Behandlung mit Harnstoff in seine Monomeren zu überführen, bzw. Iminobiotin anstelle von Biotin zu verwenden um die Bindungsaffinität herabzusetzen. Diese Verfahren sind jedoch aufwendig und mit zusätzlichen Problemen verbunden. Unter Verwendung von erfindungsgemäßen Muteinen können Affinitätsmatrices mit Bindungsaffinitäten zu Biotin hergestellt werden, die eine reversible Bindung und damit eine Rückgewinnung des Analyten ermöglichen.

Weiterhin ist es bevorzugt, die erfindungsgemäße, regenerierbare Festphase zur Bestimmung von Rezeptor/Ligand-Wechselwirkungen zu verwenden, z.B. als beschichteten Sensorchip. Die Bindung von Molekülen an diese Oberfläche kann z.B. durch Oberflächenplasmonenresonanz-Spektroskopie untersucht werden.

Die in der vorliegenden Anmeldung genannten Plasmide und Mikroorganismen wurden bei der Deutschen Sammlung von Mikroorganismen und Zellkulturen GmbH (DSM), Mascheroderweg 1B, D-30300 Braunschweig gemäß den Bestimmungen des Budapester Vertrag unter den folgenden Hinterlegungsnummern hinterlegt:

E.coli K12RM82:     DSM 5445 am 2. Oktober 1991
pUBS500:             DSM 6720 am 20. September 1991

In der Sequenzliste zeigt

SEQ ID NO. 1     die für Streptavidin kodierende Nukleotidsequenz einschließlich der Signalpeptid-kodierenden Sequenz,

SEQ ID NO. 2     die Aminosäuresequenz von Streptavidin einschließlich der Signalsequenz

SEQ ID NO. 3     die für Avidin kodierende Nukleotidsequenz einschließlich der Signalpeptid-kodierenden Sequenz,

SEQ ID NO. 4     die Proteinsequenz von Avidin einschließlich der Signalsequenz,

SEQ ID NO. 5     die Nukleotidsequenz des Primers N1,

SEQ ID NO. 6     die Nukleotidsequenz des Primers N2,

SEQ ID NO. 7     die Nukleotidsequenz des Primers N3,

SEQ ID NO. 8     die Nukleotidsequenz des Primers N4,

SEQ ID NO. 9     die Nukleotidsequenz des Primers N5,

SEQ ID NO. 10     die Nukleotidsequenz des Primers N6,

SEQ ID NO. 11     die Nukleotidsequenz des Primers N7,

SEQ ID NO. 12     die Nukleotidsequenz des Primers N8,

SEQ ID NO. 13     die Nukleotidsequenz des Primers N9,

SEQ ID NO. 14     die Nukleotidsequenz des Primers N10,

SEQ ID NO. 15     die für Core-Streptavidin gemäß WO 93/09144 kodierende Nukleotidsequenz und

SEQ ID NO. 16     die Aminosäuresequenz von Core-Streptavidin.

Figur 1      zeigt die Plasmidkarte des core-Streptavidin-Expressionsplasmids pSAM-Core.

Die folgenden Beispiele erläutern die Erfindung näher.

**Beispiel 1:**

**Konstruktion von Core-Streptavidin Mutantengenen**

Das Plasmid pSAM-core wurde gemäß WO 93/09144 hergestellt.

Der DNA-Bereich stromauf- und stromabwärts von der einzuführenden Mutation wurde bis zur nächsten singulären Restriktionsendonukleaseschnittstelle im pSAM-Core-Expressionsvektor entfernt und durch ein entsprechendes chemisch hergestelltes DNA-Segment mit der gewünschten Mutation ersetzt (DNA-Adaptor). Die Manipulation der DNA wurde dabei mit Standardmethoden durchgeführt, wie sie bei Sambrook et al., In: Molecular Cloning. A Laboratory Manual, Cold Spring Harbor Laboratory Press, Cold Spring Harbor, New York, 1989 beschrieben sind. Die verwendeten molekularbiologischen Reagenzien wurden nach den Angaben der Hersteller eingesetzt.

Die Proteinkonzentrationen der Fusionsproteine und Peptide wurde anhand der optischen Dichte (OD) bei 280 nm unter Verwendung von mittels der Aminosäuresequenzen errechneten molaren Extinktionskoeffinzienten (z.B. für Core-SA: $\varepsilon$=41820 cm$^2$/mol) bestimmt.

**Konstruktion der Streptavidin:Mutanten Einfachmutanten:**

**1.1. pSA-Leu25Trp:**

Zur Konstruktion des Leu25Trp CORE-SA Mutantengens wurde das Plasmid pSAM-CORE mit den singulär schneidenden Restriktionsendonukleasen NcoI und SalI verdaut und das ca. 2.9 kBp lange NcoI/SalI-pSAM-CORE Vektorfragment nach Isolierung mittels Agarosegelelektrophorese mit dem Leu25Trp-Adaptor ligiert. Der Leu25Trp-Adaptor wurde durch Hybridisierung aus den komplementären Oligonukleotiden N1 und N2 (Reaktionspuffer: 12,5 mmol/l Tris-HCl, pH 7,0 und 12,5 mmol/l MgCl$_2$ ; N-Konzentration: jeweils 1 pmol/60 $\mu$l) hergestellt. N1 und N2 wurden jeweils so entworfen, daß nach Hybridisierung die für die Klonierung relevanten NcoI bzw. SalI-Überhänge entstehen.

Das neuentstandene Plasmid pSA-Leu25Trp wurde durch Restriktionskartierung (Deletion der BanI-Restriktionsendonukleaseschnittstelle durch stumme Mutation im Leu25Trp-Adaptor) und die DNA-Sequenz des Adaptorbereichs durch DNA-Sequenzierung überprüft.

**Leu25Trp-Adaptor:**

```
N1: 5'- CATGGCCGAAGCTGGTATCACTGGGACCTGGTATAACCAATGGGGG-3'
N2: 5'- TCGACCCCCATTGGTTATACCAGGTCCCAGTGATACCAGCTTCGGC-3'
```

```
       NcoI-Überhang          dele.BanI                   Trp statt Leu

   5´- CATGGCCGAAGCTGGTATCACTGGGACCTGGTATAACCAATGGGGG-3´

   3´-     CGGCTTCGACCATAGTGACCCTGGACCATATTGGTTACCCCCAGCT-5´

                                                        SalI-Überhang
```

**1.2. pSA-Ser27Arg:**

Zur Herstellung des Ser27Arg CORE-SA Mutantengens wurde das Plasmid pSAM-CORE mit den singulär schneidenden Restriktionsendonukleasen NcoI und HindIII verdaut und das ca. 400 Bp lange Streptavidin-Fragment sowie das ca. 2.5 kBp pSAM-CORE Vektorfragment mittels Agarosegelelektrophorese isoliert. Die gewünschte Mutation Ser27Arg wurde mittels PCR-Technik durch Amplifikation des 400 Bp langen Fragments mit dem entsprechend entworfenem 5'-Mutageneseprimer N3 eingeführt. Das so gewonnene PCR-Produkt wurde danach mit NcoI und HindIII nachgeschnitten, mittels Agarosegelelektrophorese gereinigt und mit dem wie o.a. isolierten 2.5 kBp pSAM-CORE Vektorfragment ligiert.

Das neuentstandene Plasmid pSA-Ser27Arg wurde durch Restriktionskartierung (Deletion der Sall-Restriktionsendonukleaseschnittstelle durch Einführung der Mutation Ser27Arg) und die mutierte DNA-Sequenz des 5'-Bereichs durch DNA-Sequenzierung überprüft.

**Ser27Arg:**

Die PCR wurde mit dem 5'-Mutageneseprimer N3 (besitzt gewünschte Ser27Arg Mutation) und dem 3'-Steptavidinprimer N4 (Sequenz gegenüber pSAM-CORE unverändert) durchgeführt.

```
                 NcoI                                      Arg statt Ser
N3:  5´-GCGCCATGGCCGAAGCTGGTATCACTGGCACCTGGTATAACCAACTGGGGCGCACTTTC-3´
```

```
          HindIII
N4:  5´-CGCGAAGCTTCAGCTGTCATTAGCTA-3´
```

**1.3. pSA-Ser45Arg:**

Zur Konstruktion des Ser45Arg CORE-SA Mutantengens wurde das Plasmid pSAM-CORE mit den singulär schneidenden Restriktionsendonukleasen SacI und HindIII verdaut und das ca. 300 Bp lange Streptavidin-Fragment sowie das ca. 2.6 kBp pSAM-CORE Vektorfragment mittels Agarosegelelektzophorese isoliert. Die gewünschte Mutation Ser45Arg wurde mittels PCR-Technik durch Amplifikation des ca. 300 Bp langen Fragments mit dem entsprechend entworfenem 5'-Mutageneseprimer N5 eingeführt. Das so gewonnene PCR-Produkt wurde danach mit SacI und HindIII nachgeschnitten, mittels Agarosegelelektrophorese gereinigt und mit dem wie o.a. isolierten 2.6 kBp pSAM-CORE Vektorfragment ligiert.

Das neuentstandene Plasmid pSA-Ser45Arg wurde durch Restriktionskartierung (zusätzliche AvaII-Restriktionsendonukleaseschnittstelle durch stumme Mutation im N5-Mutageneseprimer) und die mutierte DNA-Sequenz des 5'-Bereichs durch DNA-Sequenzierung überprüft.

**Ser45Arg:**

Die PCR wurde mit dem 5'-Mutageneseprimer N5 (besitzt die gewünschte Mutation Ser45Arg) und dem 3'-Streptavidinprimer N4 (Sequenz gegenüber pSAM-CORE unverändert) durchgeführt.

```
            SacI       AvaII       Arg statt Ser
N5:  5´-GACGGAGCTCTGACTGGGACCTACGAACGCGCGGTTGGTA-3´
```

```
          HindIII
N4:  5´-CGCGAAGCTTCAGCTGTCATTAGCTA-3´
```

**1.4. pSA-Trp120A1a**

Zur Konstruktion des Trp120Ala CORE-SA Mutantengens wurde das Plasmid pSAM-CORE mit den singulär schneidenden Restriktionsendonukleasen NsiI und SpeI verdaut und das ca. 2.9 kBp lange NsiI/SpeI-pSAM-CORE Vektorfragment nach Isolierung mittels Agarosegelelektrophorese mit dem Trp120Ala-Adaptor ligiert. Der Trp120Ala-Adaptor wurde durch Hybridisierung aus den komplementären Oligonukleotiden N9 und N10 (Reaktionspuffer: 12,5 mmol/l Tris-HCl, pH 7,0 und 12,5 mmol/l MgCl$_2$; N-Konzentration: jeweils 1 pmol/60 $\mu$l) hergestellt. N9 und N10 wurden jeweils so entworfen, daß nach Hybridisierung die für die Klonierung relevanten NsiI bzw. SpeI-Überhänge entstehen.

Das neu entstandene Plasmid pSA-Trp120Ala wurde durch Restriktionskartierung (Deletion der NsiI-Restriktionsendonukleaseschnittstelle durch gewünschte Mutation im Trp120Ala-Adaptor) und die DNA-Sequenz des Adaptorbereichs durch DNA-Sequenzierung überprüft.

**Trp120A1a-Adaptor:**

    N9: 5'-GCGAAATCGACA-3'
    N10: 5'-ACTAGTGTCG ATTTCGCTGC AT-3'

                   **NsiI-Überhang   Ala            SpeI-Überhang**

       **N9:**                          **GCG**AAATCGACA

       **N10:**               **TACGT**CGC**TTTAGCTGTGATCA**

## Zweifach-Kombinationsmutanten:

### 1.5. pSA-Leu25Trp/Ser45Arg:

Zur Konstruktion des Leu25Trp/Ser45Arg CORE-SA Mutantengens wurden die Plasmide pSA-Leu25Trp und pSA-Ser45Arg mit den singulär schneidenden Restriktionsendonukleasen NcoI und SacI verdaut und das 82 Bp lange Fragment von pSA-Leu25Trp sowie das ca 2.8 kBp lange Vektorfragment von pSA-Ser45Arg mittels Agarosegelelektrophorese isoliert. Anschließend wurden beide Fragmente miteinander ligiert.

Das neuentstandene Plasmid pSA-Leu25Trp/Ser45Arg wurde mittels Restriktionskartierung (fehlende BanI-Restriktionsendonukleaseschnittstelle aus pSA-Leu25Trp und zusätzliche AvaII-Restriktionsendonukleaseschnittstelle aus pSA-Ser45Arg) und die DNA-Sequenz mittels DNA-Sequenzierung im 5'-Bereich überprüft.

### 1.6. pSA-Ser27Arg/Ser45Arg:

Zur Konstruktion des Ser27Arg/Ser45Arg CORE-SA Mutantengens wurden die Plasmide pSA-Ser27Arg und pSA-Ser45Arg mit den singulär schneidenden Restriktionsendonukleasen NcoI und SacI verdaut und das 82 Bp lange Fragment von pSA-Ser27Arg sowie das ca 2.8 kBp lange Vektorfragment von pSA-Ser45Arg mittels Agarosegelelektrophorese isoliert. Anschließend wurden beide Fragmente miteinander ligiert.

Das neuentstandene Plasmid pSA-Ser27Arg/Ser45Arg wurde mittels Restriktionskartierung (fehlende SalI-Restriktionsendonukleaseschnittstelle aus pSA-Ser27Arg und zusätzliche AvaII-Restriktionsendonukleaseschnittstelle aus pSA-Ser45Arg) und die DNA-Sequenz mittels DNA-Sequenzierung im 5.'-Bereich überprüft.

### 1.7. pSA-Ser45Trp/Leu110Trp:

Zur Konstruktion des Ser45Trp/Leu110Trp CORE-SA Mutantengens wurde das Plasmid pSA-Ser45Arg mit den singulär schneidenden Restriktionsendonukleasen SacI und NsiI verdaut und das 243 Bp lange Streptavidin-Fragment sowie das ca. 2.7 kBp lange pSA-Ser45Arg Vektorfragment mittels Agarosegelelektrophorese isoliert. Die gewünschten Mutationen Ser45Trp/Leu110Trp wurde anschließend mittels PCR-Technik durch Amplifikation des 243 Bp langen Fragments mit dem entsprechend entworfenen 5'- Mutageneseprimer N6 und dem entsprechend entworfenen 3'-Mutageneseprimer N7 eingeführt. Das so gewonnene PCR-Produkt wurde danach mit SacI und NsiI nachgeschnitten, mittels Agarosegelelektrophorese gereinigt und mit dem wie o.a. isolierten ca. 2.7 kBp pSA-Ser45Arg-Vektorfragment ligiert.

Das neuentstandene Plasmid wurde durch Restriktionskartierung (Deletion der in pSA-Ser45Arg zusätzlich durch stumme Mutation eingeführten AvaII-Schnittstelle durch Rückmutation in die ursprüngliche Sequenz sowie Deletion der HindII-Site durch die Leu110Trp-Mutation) und die gewünschten DNA-Mutationen durch DNA-Sequenzierung überprüft.

**Ser45Trp/Leu110Trp:**

Die PCR wurde mit dem 5'-Mutageneseprimer N6 (besitzt gewünschte Mutation Ser45Trp und die SacI-Restriktionsendonukleaseschnittstelle) und dem 3'-Mutageneseprimer N7 (besitzt gewünschte Mutation Leu110Trp und die NsiI-Restriktionsendonukleaseschnittstelle)

```
              SacI                         Trp statt Ser
  N6 : 5´-GACGGAGCTCTGACTGGCACCTACGAATGGGCGGTTGGTA-3´
```

```
            NsiI                        Trp statt Leu
  N7 : 5´-TTTCCATGCATTCGCTTCGGTAGTGCCGGATGTCCACAGCCACTGAGTG-3´
```

1.8. **pSA-Ser45Tyr/Leu110Trp:**

Zur Konstruktion des Ser45Tyr/Leu110Trp CORE-SA Mutantengens wurde das Plasmid pSA-Ser45Arg mit den singulär schneidenden Restriktionsendonukleasen SacI und Nsil verdaut und das 243 Bp lange Streptavidin-Fragment sowie das ca. 2.7 kBp lange pSA-Ser45Arg Vektorfragment mittels Agarosegelelektrophorese isoliert. Die gewünschten Mutationen Ser45Tyr/Leu110Trp wurde anschließend mittels PCR-Technik durch Amplifikation des 243 Bp langen Fragments mit dem entsprechend entworfenen 5'- Mutageneseprimer N8 und dem entsprechend entworfenen 3'-Mutageneseprimer N7 eingeführt. Das so gewonnene PCR-Produkt wurde danach mit SacI und Nsil nachgeschnitten, mittels Agarosegelelektrophorese gereinigt und mit dem wie o.a. isolierten ca. 2.7 kBp pSA-Ser45Arg-Vektorfragment ligiert.

Das neuentstandene Plasmid wurde durch Restriktionskartierung (Deletion der in pSA-Ser45Arg zusätzlich durch stumme Mutation eingeführten AvaII-Schnittstelle durch Rückmutation in die ursprüngliche Sequenz sowie Deletion der HindII-Site durch die Leu110Trp-Mutation) und die gewünschten DNA-Mutationen durch DNA-Sequenzierung überprüft.

**Ser45Tyr/Leu110Trp:**

Die PCR wurde mit dem 5'-Mutageneseprimer N8 (besitzt gewünschte Mutation Ser45Tyr und die SacI-Restriktionsendonukleaseschnittstelle) und dem 3'-Mutageneseprimer N7 (besitzt gewünschte Mutation Leu110Trp und die NsiI-Restriktionsendonukleaseschnittstelle)

```
              SacI                         Tyr statt Ser
  N8 :  5´-GACGGAGCTCTGACTGGCACCTACGAATATGCGGTTGGTA-3´
```

```
            NsiI                        Trp statt Leu
  N7 :  5´-TTTCCATGCATTCGCTTCGGTAGTGCCGGATGTCCACAGCCACTGAGTG-3´
```

**Dreifach-Kombinationsmutationen:**

1.9. **pSA-Leu25Trp/Ser45Trp/Leu110Trp:**

Zur Konstruktion des Leu25Trp/Ser45Trp/Leu110Trp CORE-SA Mutantengens wurden die Plasmide pSA-Leu25Trp und pSA-Ser45Trp/Leu110Trp mit den singulär schneidenden Restriktionsendonukleasen Sall und HindIII verdaut und das 352 Bp lange Fragment von pSA-Ser45Trp/- Leu110Trp sowie das ca 2.6 kBp lange Vektorfragment von pSA-Leu25Trp mittels Agarosegelelektrophorese isoliert. Anschließend wurden beide Fragmente miteinander ligiert.

Das neuentstandene Plasmid pSA-Leu25Trp/Ser45Trp/Leu110Trp wurde mittels Restriktionskartierung (fehlende HindII-Restriktionsendonukleaseschnittstelle aus pSA-Ser45Trp/Leu110Trp und fehlende BanI-Restriktionsendonukleaseschnittstelle aus pSA-Leu25Trp) und mittels DNA-Sequenzierung überprüft.

1.10. **pSA-Leu25Trp/Ser45Tyr/Leu110Trp:**

Zur Konstruktion des Leu25Trp/Ser45Tyr/Leu110Trp CORE-SA Mutantengens wurden die Plasmide pSA-Leu25Trp und pSA-Ser45Tyr/Leu110Trp mit den singulär schneidenden Restriktionsendonukleasen SalI und HindIII verdaut und das 352 Bp lange Fragment von pSA-Ser45Tyr/- Leu110Trp sowie das ca 2.6 kBp lange Vektorfragment von pSA-Leu25Trp mittels Agarosegelelektrophorese isoliert. Anschließend wurden beide Fragmente miteinander ligiert.
Das neuentstandene Plasmid pSA-Leu25Trp/Ser45Trp/Leu110Trp wurde mittels Restriktionskartierung (fehlende HindII-Restriktionsendonukleaseschnittstelle aus pSA-Ser45Trp/Leu110Trp und fehlende BanI-Restriktionsendonukleaseschnittstelle aus pSA-Leu25Trp) und die DNA-Sequenz mittels DNA-Sequenzierung überprüft.

1.11. **pSA-Ser27Arg/Ser45Arg/Leu110Trp:**

Zur Konstruktion des Ser27Arg/Ser45Arg/Leu110Trp CORE-SA Mutantengens wurden die Plasmide pSA-Ser27Arg/Ser45Arg und pSA-Ser45Trp/- Leu110Trp mit den singulär schneidenden Restriktionsendonukleasen KpnI und HindIII verdaut und das 218 Bp lange Fragment von pSA-Ser45Trp/Leu110Trp sowie das ca 2.7 kBp lange Vektorfragment von pSA-Ser27Arg/Ser45Arg mittels Agarosegelelektrophorese isoliert. Anschließend wurden beide Fragmente miteinander ligiert.
Das neuentstandene Plasmid pSA-Ser27Arg/Ser45Arg/Leu110Trp wurde mittels Restriktionskartierung (fehlende HindII-Restriktionsendonukleaseschnittstellen aus pSA-Ser27Arg/Ser45Arg und pSA-Ser45Trp/Leu110Trp und zusätzliche AvaII-Restriktionsendonukleaseschnittstelle aus pSA-Ser27Arg/Ser45Arg) und die DNA-Sequenz mittels DNA-Sequenzierung überprüft.

1.12. **pSA-Ser27Arg/Ser45Arg/Trp120A1a**

Zur Konstruktion des Ser27Arg/Ser45Arg/Trp120Ala-CORE-SA Mutantengens wurde das Plasmid pSA-Ser27Arg/Ser45Arg mit den singulär schneidenden Restriktionsendonukleasen NsiI und SpeI verdaut und das ca. 2.9 kBp lange NsiI/SpeI -pSAM-CORE Vektorfragment nach Isolierung mittels Agarosegelelektrophorese mit dem Trp120Ala-Adaptor ligiert. Der Trp120Ala-Adaptor wurde durch Hybridisierung aus den komplementären Oligonukleotiden N9 und N10 (Reaktionspuffer: 12,5 mmol/l Tris-HCl, pH 7,0 und 12,5 mmol/l MgCl2; N-Konzentration: jeweils 1 pmol/60µl) hergestellt. N9 und N10 wurden jeweils so entworfen, daß nach Hybridisierung die für die Klonierung relevanten NsiI bzw. SpeI-Überhänge entstehen.
Das neuentstandene Plasmid pSA-Ser27Arg/Ser45Arg/Trp120Ala wurde durch Restriktionskartierung (Deletion der NsiI-Restriktionsendonukleaseschnittstelle durch gewünschte Mutation im Trp120Ala-Adaptor) und die DNA-Sequenz des Adaptorbereichs durch DNA-Sequenzierung überprüft.

**Beispiel** 2:

**Charakterisierung von gereinigten inaktiven Core-Streptavidin Muteinen aus E.coli**

**2.1. Expression**

Die Expression der Core-Streptavidin Muteine in E. coli, die Expressionsanalyse, die Präparation sowie die Reinigung von naturierten inaktiven Core-Streptavidin Muteinen erfolgte wie in der EP-A-0 612 325 für rekombinantes Core-Streptavidin beschrieben. Bei der Doppelmutante SA-Ser27Arg/Ser45Arg und der Dreifachmutante SA-Ser27Arg/Ser45Arg/Leu110Trp wurde anstelle von Q-Sepharose zur Aufreinigung eine in 20 mM Na-Acetat, pH 5,0 äquilibrierte S-Sepharose verwendet. Die Elution erfolgte in 20 mM Na-Acetat, pH 5,0 mit 350 mM NaCl.
Die SA-Mutante Trp120Ala zeigte gegenüber den anderen Mutanten eine verringerte Säurestabilität, was auf eine unrichtige strukturelle Faltung sowie auf Störungen des Proteinrückgrats hinwies.

**2.2. SDS-PAGE/Isoelektrische Fokussierung (IF)**

Die Homogenität und Reinheit der naturierten gereinigten inaktiven Core-Streptavidin Muteinen wurde durch SDS-PAGE (Laemmli, Nature 227 (1970) 680 - 685) und Isoelektrische Fokussierung (Bark et al., J. Forensic Sci. Soc. 16 (1976) 115 - 120) untersucht.

EP 0 799 890 B1

Die naturierten gereinigten inaktiven Core-Streptavidin Muteine waren homogen (einbandig) in der SDS-PAGE-(Molekulargewicht ca. 13500 Da) und in den IF-Gelen mit einer Reinheit von > 98%.

### Beispiel 3

### Bestimmung der Affinität rekombinanter Streptavidin Mutanten

Die Affinität der Streptavidin-Muteine zu Biotinderivaten (Konstanten $K_{on}$, $K_{off}$ und $K_A$) wurde mit dem BIAcore-System der Fa.

Pharmacia im Vergleich zu Core-Streptavidin (Wildtyp-Streptavidin) bestimmt. Hierzu wurden die jeweiligen Streptavidin-Proben an der Oberfläche von Biosensor-Chips (CM5 Biosensor) immobilisiert. Die Oberflächenbeladung wurde so gewählt, daß ein Meßsignal von 500 bis 2000 Resonanzunits (rU) erzeugt wurde. Die Assoziationskinetiken wurden bei 25°C und 6 Konzentrationen (12,5, 25, 50, 100, 200 und 400 nmol/l) eines monobiotinylierten Fab'-Antikörperfragments für jeweils 3 min aufgenommen. Um den Einfluß der Rückbindung des Antikörpers zu untersuchen, wurden die Dissoziationskinetiken in Abwesenheit und Gegenwart von 10 μg/ml Core-Streptavidin verglichen. Die Spezifität der Bindung wurde durch einen Versuch mit dem gleichen Antikörper in nichtbiotinylierter Form (negative Kontrolle) gezeigt.

Die Bindungskonstanten wurden sowohl von den gereinigten Streptavidin-Muteinen als auch von den zu einem Polymer vernetzten Streptavidin-Muteinen (Streptavidin-Mutein-Thermo-Rinderserumalbumin-Konjugate, Herstellung: EP-A-0 269 092) bestimmt. Die Ergebnisse des Experiments sind in Tabelle 1 gezeigt.

**Tabelle 1**

| Streptavidin Wildtyp/Mutein | $K_{on}$ 1/mol·s | $K_{off}$ 1/s | $K_A$ 1/mol | t/2 diss min | Regenerierbarkeit |
|---|---|---|---|---|---|
| Wildtyp | $2,2.10^5$ | $< 10^{-5}$ | $> 10^{10}$ | > 20 h | nein |
| SA-Ser27Arg/ Ser45Arg (1) | $1,3·10^5$ | $3,1·10^{-3}$ | $4,2·10^7$ | 3.7 | ja |
| | $8,5·10^4$ | $4,1·10^{-3}$ | $2.1·10^7$ | 2,8 | |
| SA-Ser45Trp/ Leu110Trp | $1,4·10^5$ | $3,3·10^{-4}$ | $6·10^8$ | 40,8 | ja |
| SA-Ser45Tyr/ Leu110Trp | $1,3·10^5$ | $5,4·10^{-4}$ | $2,7·10^5$ | 22,6 | ja |
| SA-Leu25Trp/ Ser45Arg | $7,9·10^4$ | $2,2·10^{-4}$ | $3,6·10^8$ | 51,8 | ja |
| SA-Ser27Arg/ Ser45Arg TRSA-Konjugat | $1,0·10^5$ | $3,0·10^{-3}$ | $3,3·10^7$ | 3,9 | ja |
| SA-Leu25Trp/ Ser45Trp/ Leu110Trp | $4,9·10^4$ | $8,7·10^{-4}$ | $7,0·10^7$ | 13,4 | ja |
| SA-Leu25Trp/ Ser45Tyr/ Leu110Trp | $5,4·10^4$ | $1,3·10^{-3}$ | $4,6·10^7$ | 8,7 | ja |
| SA-Ser27Arg/ Ser45Arg/ Leu110Trp | $4, 5·10^4$ | $5,9·10^{-3}$ | $7,77·10^6$ | 2,0 | ja |
| SA-Trp120Ala | $6,7·10^4$ | $2,3·10^{-4}$ | $2,9·10^9$ | 49,4 | ja |
| SA-Ser27Arg/ Ser45Arg/ Trp120/Ala | $1,0·10^5$ | $5,2·10^{-3}$ | $2,0·10^7$ | 2,2 | ja |
| (1) Daten aus 2 Produktchargen | | | | | |

### Beispiel 4

### Entstörexperiment

Es wurde ein Enzymuntest auf Anti-HCV-Antikörper durchgeführt und die Reduzierung von Teststörungen durch Zugabe der Streptavidin-Muteine SA-Ser27Arg/Ser45Arg (1), SA-Ser45Trp/Leu110Trp (2) und SA-Ser45Tyr/Leu110Trp (3) bestimmt.

Der Test wurde als Zwei-Schritt-Sandwichassay mit einer Streptavidin-Festphase durchgeführt. Im ersten Schritt wurden biotinylierte HCV-Peptide (EP-A-0 582 243, EP-A-0 484 787) bzw. Polypeptide (EP-A-0 696 640) plus Probe zugesetzt. Als zweiter Schritt wurde eine Reaktion der Festphasen-gebundenen Antikörper mit einem Anti-Human-IgG-Peroxidase-Konjugat durchgeführt. Anschließend wurde eine Indikatorreaktion mit ABTS als Substrat durchgeführt.

Die Durchführung des Tests war wie folgt:

Inkubationspuffer: Na-Phosphat 40 mmol/l pH: 7,4
NaCl 7,1 g/L
Konservierungsmittel (z.B. Chloracetamid)
Plasma-Diagnostic-Base 200 g/L (Armour Pharmaceuticals Company, USA)
+ HCV-Peptide aus der Core, NS4 und NS3 Region und rekombinantes NS3 Polypeptid (biotinyliert)
± 25 µg/ml Streptavidin-Mutein unkonjugiert bzw. 75µg/ml als Konjugat mit Thermo-RSA
± 25 µg/ml Core-Streptavidin (WT)

Konjugatpuffer: Na-Phosphat 40 mmol/l pH: 7,4
NaCl 7,1 g/l
Konservierungsmittel (z.B. Chloracetamid)
Rinderserumalbumin 1 g/L
Rinder IgG 4 g/L
Triton X 100 1 g/L
Anti-human IgG-POD 15 U/L

Inkubationszeiten: 1. Schritt: 1 Stunde (Probe + Inkubationspuffer)
2. Schritt: 1 Stunde (+ Konjugatpuffer)
3. Schritt: 1 Stunde (Substratreaktion mit ABTS)
Testdurchführung am ES 600 bei 25°C
Messung der Substratlösung bei 422 nm

Proben: 5 falsch positive Anti-HCV-Negativproben (Störproben)
6 positive Anti HCV-Proben (Kontrolle)

Volumina: Probe 20 µl, alle anderen Reagenzien jeweils 500 µl

Die am Analysegerät ES 600 bei 422 nm gemessenen Extinktionswerte sind in den nachfolgenden Tabellen 2 und 3 angegeben. Die Ergebnisse zeigen, daß der Zusatz der Streptavidin-Muteine eine sehr starke Signalabnahme bei den Negativserumproben, aber keine oder nur eine sehr geringe Signalabnahme bei den Positivserumproben bewirkt. Das Streptavidinmutein eignet sich daher hervorragend als EntstBrungsreagenz.

Tabelle 2

| Proben | Ohne Zusatz im Puffer | Mit SA-Mutein (1) im Puffer | Mit SA-Mutein (1) Konjugat im Puffer | Mit Core-SA (wt) im Puffer | % Signalabnahme nach Zugabe | |
| | | | | | SA-Mutein | SA-Mutein-Konjugat |
|---|---|---|---|---|---|---|
| HCV-Negativserum 1 | 0,324 | 0,174 | 0,175 | 0,002 | 46% | 46% |
| HCV-Negativserum 2 | 0,838 | 0,221 | 0,109 | 0.652 | 74% | 87% |
| HCV-Negativserum 3 | 1,675 | 1,025 | 0,538 | 0,256 | 39% | 68% |
| HCV-Negativserum 4 | 2,854 | 1,413 | 1,283 | 0,660 | 50% | 55% |
| HCV-Negativserum 5 | 4,935 | 2,661 | 2,511 | 1,403 | 46% | 49% |
| HCV Positivserum 1 | 5,152 | 5,219 | 4,722 | 0,061 | -1% | 8% |

(fortgesetzt)

| Proben | Ohne Zusatz im Puffer | Mit SA-Mutein (1) im Puffer | Mit SA-Mutein (1) Konjugat im Puffer | Mit Core-SA (wt) im Puffer | % Signalabnahme nach Zugabe | |
|---|---|---|---|---|---|---|
| | | | | | SA-Mutein | SA-Mutein-Konjugat |
| HCV Positivserum 2 | 1,051 | 1,235 | 0,896 | 0,023 | -18% | 15% |
| HCV Positivserum 3 | 2,730 | 2,585 | 2,385 | 0,027 | 5% | 13% |
| HCV Positivserum 4 | 5,030 | 5,111 | 4,629 | 0.008 | -2% | 8% |
| HCV Positivserum 5 | 2,244 | 2,216 | 1,782 | 0,003 | 1% | 21% |
| HCV Positivserum 6 | 4,920 | 4,612 | 4,523 | 0,006 | 6% | 8% |

Tabelle 3

| Proben | Ohne Zusatz im Puffer | Mutein (2) im Puffer | Mutein (3) im Puffer | Mit Core-SA (wt) im Puffer | % Signalabnahme nach Zugabe SA-Mutein | |
|---|---|---|---|---|---|---|
| | | | | | (2) | (3) |
| HCV-Negativserum 1 | 0,221 | 0,105 | 0,052 | 0,452 | 52% | 76% |
| HCV-Negativserum 2 | 1,002 | 0,238 | 0,248 | 0,652 | 76% | 75% |
| HCV-Netativserum 3 | 2,195 | 1,003 | 1,118 | 0,494 | 54% | 49% |
| HCV-Negativserum 4 | 3,143 | 1,606 | 1,643 | 0,660 | 49% | 48% |
| HCV-Negativaerum 5 | 6,419 | 2,961 | 3,222 | 2,518 | 54% | 50% |
| HCV-Positivserum 1 | 5,349 | 5,604 | 5,199 | 0,016 | -5% | 3% |
| HCV-Positivserum 2 | 0,949 | 1,050 | 0,954 | 0,012 | -11% | -1% |
| MC:V-Positivserum 3 | 2,862 | 2,619 | 2,664 | 0,003 | 8% | 7 |
| HCV-Positivserum 4 | 0,549 | 0,516 | 0,515 | 0,015 | 6% | 6 |
| HCV-Positivserum 5 | 4,287 | 4,089 | 4,727 | 0,003 | 5% | -10% |

### Beispiel 5

### Verwendung von Strentavidin-Muteinen als Affinitätsadsorbens

### 5.1 Fixierung von SA-Mutein an Spherosil-NH$_2$

Spherosil-NH$_2$ (Boehringer Mannheim, Bestell-Nummer: 576590) wurde mit der dreifachen Menge 10 % (w/v) Glutardialdehyd aktiviert und dann mit 7 Volumina destilliertem Wasser gewaschen. Das Streptavidin-Mutein wurde an die entstandenen freien Aldehydgruppen mittels seiner freien Aminogruppen gekoppelt. Die verwendete SA-Mutein-Konzentration betrug 2 mg Streptavidin-Mutein pro ml aktiviertes Spherosil-NH$_2$ Gel. Das nicht an Spherosil gebundene Mutein wurde mit PBS-Puffer (50 mM K-Phosphat, pH 7,5, 150 mM NaCl) ausgewaschen.

Anschließend wurde das Adsorbens mit biotinylierten Substanzen beladen. Nichtbiotinylierte Substanzen wurden nicht gebunden und konnten somit von den biotinylierten Substanzen leicht abgetrennt werden. Auch eine Auftrennung biotinylierter Substanzen entsprechend dem Biotinylierungsgrad (Anzahl der Biotingruppen pro Molekül) konnte durchgeführt werden.

Die ah der Säule gebundenen biotinylierten Substanzen konnten mit einem Puffer pH < 4,5 oder/und durch Zugabe chaotroper Substanzen, wie z.B. Guanidiniumhydrochlorid, oder/und durch Zugabe von Biotin oder Biotinanaloga eluiert werden. Die Auftrennung von biotinylierten Substanzen entsprechend dem Biotinylierungsgrad erfolgte bevorzugt mittels eines Biotin- oder Biotinanalogon-Gradienten.

### 5.2 Fixierung von SA-Mutein an andere Träger

Eine Fixierung von Streptavidin-Muteinen an andere Chromatographiematerialien ist über alle für natives Streptavidin bekannten Verfahren möglich.

### 5.3. Beladung eines SA-Mutein Adsorbers mit biotinyliertem Rinderserum-Albumin (Bi-RSA)

Der in Beispiel 5.1. hergestellte Sperosil-NH$_2$ SA-Mutein Adsorber wurde in PBS-Puffer (50 mM K-Phosphat, pH 7,5, 150 mM NaCl) äquilibriert und in eine Chromatographiesäule überführt. Dann wurden 2 mg Bi-RSA in PBS pro ml SA-Mutein Adsorber aufgetragen. Nichtgebundenes Protein wurde mit 2 Säulenvolumina PBS ausgewaschen. Die Elution erfolgte in 50 mM Ammoniumacetat, pH 3,0 oder/und einem Gradienten von 0 bis 10 mM Iminobiotin oder Biotin.

### 5.4. Beladung eines SA-Mutein Adsorbers mit einem biotinylierten Fab-Antikörperfragment

Der in Beispiel 5.1. hergestellte SA-Mutein Adsorber wurde in PBS-Puffer mit 500 mM Ammoniumsulfat (PBS + AS-Puffer) äquilibriert und in eine Chromatographiesäule überführt. Dann wurden 2 mg Antikörperfragment pro ml SA-Mutein-Adsorber in PBS + AS-Puffer aufgetragen. Nichtgebundenes Protein wurde mit 2 Säulenvolumina PB+AS-Puffer ausgewaschen. Die Elution erfolgte mit PBS-Puffer, pH 7,2 und einem Gradienten von 0 bis 10 mM Biotin.

**Patentansprüche**

1.  Biotin-bindefähiges Polypeptid, ausgewählt aus Muteinen von Streptavidin,
    **dadurch gekennzeichnet,**
    **daß** das Mutein (a) sich um mindestens eine Aminosäure vom nativen Polypeptid unterscheidet, wobei mindestens eine der Aminosäuren an den Positionen Leu25, Ser27, Tyr43, Ser45, Val47, Gly48, Ser88. Thr90, Leu110 oder/und Asp 128 durch eine andere Aminosäure ausgetauscht ist, (b) eine Bindungsaffinität zu Biotin von weniger als $10^{10}$ l/mol aufweist.

2.  Polypeptid nach Anspruch 1
    **dadurch gekennzeichnet,**
    **daß** mindestens eine der Aminosäuren Leu25, Ser27, Ser45 und Leu110 durch eine Aminosäure ausgewählt aus Arg, Trp, Tyr, Phe und His ausgetauscht ist.

3.  Polypeptid nach einem der vorhergehenden Ansprüche,
    **dadurch gekennzeichnet,**
    **daß** es als polymeres Konjugat vorliegt.

4.  Polypeptid nach einem der vorhergehenden Ansprüche,
    **dadurch gekennzeichnet,**
    **daß** das Mutein die Fähigkeit aufweist, Dimere oder/und Tetramere zu bilden.

5.  Nukleinsäure,
    **dadurch gekennzeichnet,**
    **dass** sie für ein Polypeptid nach einem der Ansprüche 1 bis 4 kodiert.

6.  Vektor,
    **dadurch gekennzeichnet,**
    **dass** er mindestens eine Kopie einer Nukleinsäure nach Anspruch 5 enthält.

7.  Zelle,
    **dadurch gekennzeichnet,**
    **dass** sie mit einem Vektor nach Anspruch 6 transformiert ist, und die Nukleinsäure nach Anspruch 5 exprimiert.

8.  Verwendung eines Polypeptids nach einem der Ansprüche 1 bis 4 als Entstörungsreagenz für Assays, die das Bindepaar Streptavidin-Biotin als Testkomponenten enthalten.

9.  Entstörungsreagenz zur Verringerung oder/und Vermeidung von unspezifischen Wechselwirkungen in einem Verfahren zum Nachweis eines Analyten,
    **dadurch gekennzeichnet,**
    **dass** es ein Mutein nach einem der Ansprüche 1-4 enthält.

10. Verfahren zum qualitativen oder/und quantitativen Nachweis eines Analyten in einer Testprobe, umfassend die Verwendung des spezifischen Bindepaares Streptavidin-Biotin, wobei man der Testprobe ein Polypeptid nach einem der Ansprüche 1 bis 4 zugibt.

11. Testkit zum qualitativen oder/und quantitativen Nachweis eines Analyten in einer Testprobe enthaltend ein Biotin-bindefähiges Polypeptid sowie weitere Komponenten des jeweiligen Assays und zusätzlich ein Entstörungsreagenz nach Anspruch 9.

12. Verwendung eines Biotin-bindefähigen Polypeptids, ausgewählt aus Muteinen von Streptavidin, als regenerierbares System zur Bindung von Biotin,
    **dadurch gekennzeichnet,**
    **dass** das Mutein (a) sich um mindestens eine Aminosäure vom native Polypeptid der SEQ ID NO: 2 unterscheidet, wobei mindestens eine der Aminosäuren an den Positionen Leu25, Ser27, Tyr43, Ser45, Val47, Gly48, Ser88, Thr90, Leu110 oder/und Asp128 durch eine andere Aminosäure ausgetauscht ist, (b) eine Bindungsaffinität zu Biotin von $10^5$-$10^{11}$l/mol aufweist.

**13.** Verwendung nach Anspruch 12,
**dadurch gekennzeichnet,**
**dass** die Regenerierung des Systems durch Verringerung des pH-Werts auf < 4,5 oder/und durch Zugabe einer chaotropen Substanz erfolgt.

**14.** Biotin-bindefähige regenierbare Festphase,
**dadurch gekennzeichnet,**
**dass** sie mit einem Mutein von Streptavidin beschichtet ist, das (a) sich um mindestens eine Aminosäure vom nativen Polypeptid der SEQ ID NO: 2 unterscheidet, wobei mindestens eine der Aminosäuren an den Positionen Leu25, Ser27, Tyr43, Ser45, Val47, Gly48, Ser88, Thr90, Leu110 oder/und Asp128 durch eine andere Aminosäure ausgetauscht ist, (b) eine Bindungsaffinität zu Biotin von weniger als $10^{10}$ l/mol aufweist.

**Claims**

**1.** Polypeptide capable of binding to biotin selected from muteins of streptavidin,
**characterized in that**
the mutein (a) differs from the native polypeptide by at least one amino acid where at least one of the amino acids at positions Leu25, Ser27, Tyr43, Ser45, Val47, Gly48, Ser88, Thr90, Leu110 or/and Asp128 is substituted by another amino acid, (b) has a binding affinity to biotin of less than $10^{10}$ l/mol.

**2.** Polypeptide according to claim 1,
**characterized in that**
at least one of the amino acids Leu25, Ser27, Ser45 and Leu110 is substituted by an amino acid selected from Arg, Trp, Tyr, Phe and His.

**3.** Polypeptide according to one of the previous claims,
**characterized in that**
it is present as a polymeric conjugate.

**4.** Polypeptide according to one of the previous claims,
**characterized in that**
the mutein has the ability to form dimers or/and tetramers.

**5.** Nucleic acid
**characterized in that**
it codes for a polypeptide according to one of the claims 1 to 4.

**6.** Vector
**characterized in that**
it contains at least one copy of a nucleic acid according to claim 5.

**7.** Cell,
**characterized in that**
it is transformed with a vector according to claim 6 and expresses the nucleic acid according to claim 5.

**8.** Use of a polypeptide according to one of the claims 1 to 4 as an interference elimination reagent for assays which contain the binding pair streptavidin-biotin as test components.

**9.** Interference elimination reagent for reducing or/and avoiding unspecific interactions in a method for the detection of an analyte,
**characterized in that**
it contains a mutein according to one of the claims 1 to 4.

**10.** Method for the qualitative or/and quantitative detection of an analyte in a test sample comprising the use of the specific binding pair streptavidin-biotin, wherein a polypeptide according to one of the claims 1 to 4 is added to the test sample.

11. Test kit for the qualitative or/and quantitative detection of an analyte in a test sample containing a polypeptide capable of binding to biotin as well as further components of the respective assay and additionally an interference elimination reagent according to claim 9.

12. Use of a polypeptide capable of binding to biotin selected from muteins of streptavidin as a regeneratable system for binding biotin,
**characterized in that**
the mutein (a) differs from the native polypeptide of SEQ ID NO: 2 by at least one amino acid where at least one of the amino acids at positions Leu25, Ser27, Tyr43, Ser45, Va147, Gly48, Ser88, Thr90, Leu110 or/and Asp128 is substituted by another amino acid, (b) has a binding affinity for biotin of less than $10^5$ - $10^{11}$ l/mol.

13. Use according to claim 12,
**characterized in that**
the system is regenerated by lowering the pH value to < 4.5 or/and by adding a chaotropic substance.

14. Regeneratable solid phase capable of binding to biotin,
**characterized in that**
it is coated with a mutein of streptavidin, which (a) differs from the native polypeptide of SEQ ID NO: 2 by at least one amino acid where at least one of the amino acids at positions Leu25, Ser27, Tyr43, Ser45, Va147, Gly48, Ser88, Thr90, Leu110 or/and Asp128 is substituted by another amino acid, (b) has a binding affinity for biotin of less than $10^{10}$ l/mol.

**Revendications**

1. Polypeptide capable de lier la biotine choisi parmi les mutéines de streptavidine **caractérisé en ce que** la mutéine (a) diffère du polypeptide natif d'au moins un aminoacide, où au moins l'un des aminoacides aux positions Leu25, Ser27, Tyr43, Ser45, Va147, Gly48, Ser88, Thr90, Leu110 et/ou Asp128 est remplacé par un autre aminoacide, (b) présente une affinité de liaison pour la biotine inférieure à $10^{10}$ l/mol.

2. Polypeptide selon la revendication 1 **caractérisé en ce qu'**au moins l'un des aminoacides Leu25, Ser27, Ser45 et Leu110 est remplacé par un aminoacide choisi parmi Arg, Trp, Tyr, Phe et His.

3. Polypeptide selon l'une des revendications précédentes **caractérisé en ce qu'**il est sous forme de conjugué polymère.

4. Polypeptide selon l'une des revendications précédentes **caractérisé en ce que** la mutéine présente la capacité à former des dimères et/ou des tétramères.

5. Acide nucléique **caractérisé en ce qu'**il code un polypeptide selon l'une des revendications 1 à 4.

6. Vecteur **caractérisé en ce qu'**il contient au moins une copie d'un acide nucléique selon la revendication 5.

7. Cellule **caractérisée en ce qu'**elle est transformée avec un vecteur selon la revendication 6 et exprime l'acide nucléique selon la revendication 5.

8. Utilisation d'un polypeptide selon l'une des revendications 1 à 4 comme réactif anti-perturbation pour des essais qui contiennent la paire à liaison streptavidine-biotine comme composants de test.

9. Réactif anti-perturbation pour réduire et/ou éviter des interactions non spécifiques dans un procédé de mise en évidence d'un analyte **caractérisé en ce qu'**il contient une mutéine selon l'une des revendications 1-4.

10. Procédé de mise en évidence qualitative et/ou quantitative d'un analyte dans un échantillon test, comprenant l'utilisation de la paire à liaison spécifique streptavidine-biotine, où on ajoute à l'échantillon test un polypeptide selon l'une des revendications 1 à 4.

11. Kit de test pour la mise en évidence qualitative et/ou quantitative d'un analyte dans un échantillon test contenant un polypeptide capable de lier la biotine ainsi que d'autres composants de l'essai respectif et en outre un réactif anti-perturbation selon la revendication 9.

**12.** Utilisation d'un polypeptide capable de lier la biotine, choisi parmi les mutéines de streptavidine, comme système régénérable pour lier la biotine, **caractérisée en ce que** la mutéine (a) diffère du polypeptide natif de SEQ ID NO : 2 d'au moins un aminoacide, où au moins l'un des aminoacides aux positions Leu25, Ser27, Tyr43, Ser45, Va147, Gly48, Ser88, Thr90, Leu110 et/ou Asp128 est remplacé par un autre aminoacide, (b) présente une affinité de liaison pour la biotine de $10^5$-$10^{11}$ l/mol.

**13.** Utilisation selon la revendication 12 **caractérisée en ce que** la régénération du système a lieu par réduction du pH à < 4,5 et/ou par addition d'une substance chaotrope.

**14.** Phase solide régénérable capable de lier la biotine **caractérisée en ce qu'**elle est recouverte d'une mutéine de streptavidine qui (a) diffère du polypeptide natif de SEQ ID NO : 2 d'au moins un aminoacide, où au moins l'un des aminoacides aux positions Leu25, Ser27, Tyr43, Ser45, Va147, Gly48, Ser88, Thr90, Leu110 et/ou Asp128 est remplacé par un autre aminoacide, (b) présente une affinité de liaison pour la biotine inférieure à $10^{11}$ l/mol.

Fig.1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 260903 A
- US 4931385 A
- US 5051356 A
- EP 0525916 A
- EP 0163312 A
- DE 4407423 A
- DE 4434093 A

- WO 9309144 A
- EP 0269092 A
- EP 0612325 A
- EP 0582243 A
- EP 0484787 A
- EP 0696640 A

**In der Beschreibung aufgeführte Nicht-Patentliteratur**

- **Robertson et al.** *J. of Immun. Med.,* 1985, vol. 26, 195
- **Chilkoti et al.** *Proc. Natl. Acad. Sci. USA,* 1995, vol. 92, 1754-1758
- **Sano ; Cantor.** *Proc. Natl. Acad. Sci. USA,* 1995, vol. 92, 3180-3184

- **Sambrook et al.** Molecular Cloning. A Laboratory Manual. Cold Spring Harbor Laboratory Press, 1989
- **Laemmli.** *Nature,* 1970, vol. 227, 680-685
- **Bark et al.** *J. Forensic Sci. Soc.,* 1976, vol. 16, 115-120

SEQUENZPROTOKOLL

(1) ALLGEMEINE ANGABEN:

(i) ANMELDER:

(A) NAME: Boehringer Mannheim GmbH
(B) STRASSE: Sandhofer Str. 112-132
(C) ORT: Mannheim
(E) LAND: DE
(F) POSTLEITZAHL: 68305

(ii) BEZEICHNUNG DER ERFINDUNG: Rekombinante inaktive Core-Streptavidin Muteine

(iii) ANZAHL DER SEQUENZEN: 16

(iv) COMPUTER-LESBARE FASSUNG:

(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(2) ANGABEN ZU SEQ ID NO: 1:

(i) SEQUENZKENNZEICHEN:

(A) LÄNGE: 638 Basenpaare
(B) ART: Nucleotid
(C) STRANGFORM: Einzelstrang
(D) TOPOLOGIE: linear

(ix) MERKMAL:

(A) NAME/SCHLÜSSEL: CDS
(B) LAGE:50..598

(ix) MERIOKAL:

(A) NAME/SCHLÜSSEL: sig_peptide
(B) LAGE:50..121

(ix) MERKMAL:

(A) NAME/SCHLÜSSEL: mat_peptide
(B) LAGE:122..598

(xi) SEQUENZBESCHREIBUNG: SEQ ID NO: 1:

```
CCCTCCGTCC  CCGCCGGGCA  ACAACTAGGG  AGTATTTTTC  GTGTCTCAC ATG CGC      55
                                                          Met Arg
                                                          -24

AAG ATC GTC GTT GCA GCC ATC GCC GTT TCC CTG ACC ACG GTC TCG ATT      103
Lys Ile Val Val Ala Ala Ile Ala Val Ser Leu Thr Thr Val Ser Ile
        -20             -15                 -10

ACG GCC AGC GCT TCG GCA GAC CCC TCC AAG GAC TCG AAG GCC CAG GTC      151
Thr Ala Ser Ala Ser Ala Asp Pro Ser Lys Asp Ser Lys Ala Gln Val
        -5               1               5                   10

TCG GCC GCC GAG GCC GGC ATC ACC GGC ACC TGG TAC AAC CAG CTC GGC      199
Ser Ala Ala Glu Ala Gly Ile Thr Gly Thr Trp Tyr Asn Gln Leu Gly
                    15              20              25
```

```
TCG ACC TTC ATC GTG ACC GCG GGC GCC GAC GGC GCC CTG ACC GGA ACC     247
Ser Thr Phe Ile Val Thr Ala Gly Ala Asp Gly Ala Leu Thr Gly Thr
            30                      35                  40

TAC GAG TCG GCC GTC GGC AAC GCC GAG AGC CGC TAC GTC CTG ACC GGT     295
Tyr Glu Ser Ala Val Gly Asn Ala Glu Ser Arg Tyr Val Leu Thr Gly
            45                      50                  55

CGT TAC GAC AGC GCC CCG GCC ACC GAC GGC AGC GGC ACC GCC CTC GGT     343
Arg Tyr Asp Ser Ala Pro Ala Thr Asp Gly Ser Gly Thr Ala Leu Gly
        60                      65                  70

TGG ACG GTG GCC TGG AAG AAT AAC TAC CGC AAC GCC CAC TCC GCG ACC     391
Trp Thr Val Ala Trp Lys Asn Asn Tyr Arg Asn Ala His Ser Ala Thr
    75                  80                  85                  90

ACG TGG AGC GGC CAG TAC GTC GGC GGC GCC GAG GCG AGG ATC AAC ACC     439
Thr Trp Ser Gly Gln Tyr Val Gly Gly Ala Glu Ala Arg Ile Asn Thr
                95                  100                 105

CAG TGG CTG CTG ACC TCC GGC ACC ACC GAG GCC AAC GCC TGG AAG TCC     487
Gln Trp Leu Leu Thr Ser Gly Thr Thr Glu Ala Asn Ala Trp Lys Ser
            110                 115                 120

ACG CTG GTC GGC CAC GAC ACC TTC ACC AAG GTG AAG CCG TCC GCC GCC     535
Thr Leu Val Gly His Asp Thr Phe Thr Lys Val Lys Pro Ser Ala Ala
            125                 130                 135

TCC ATC GAC GCG GCG AAG AAG GCC GGC GTC AAC AAC GGC AAC CCG CTC     583
Ser Ile Asp Ala Ala Lys Lys Ala Gly Val Asn Asn Gly Asn Pro Leu
        140                 145                 150

GAC GCC GTT CAG CAG TAGTCGCGTC CCGGCACCGG CGGGTGCCGG GACCTCGGCC      638
Asp Ala Val Gln Gln
155
```

(2) ANGABEN ZU SEQ ID NO: 2:

    (i) SEQUENZKENNZEICHEN:

        (A) LÄNGE: 183 Aminosäuren
        (B) ART: Aminosäure
        (D) TOPOLOGIE: linear

    (ii) ART DES MOLEKÜLS: Protein

    (xi) SEQUENZBESCHREIBUNG: SEQ ID NO: 2:

```
Met Arg Lys Ile Val Val Ala Ala Ile Ala Val Ser Leu Thr Thr Val
-24          -20              -15                     -10

Ser Ile Thr Ala Ser Ala Ser Ala Asp Pro Ser Lys Asp Ser Lys Ala
            -5                   1                   5

Gln Val Ser Ala Ala Glu Ala Gly Ile Thr Gly Thr Trp Tyr Asn Gln
        10                  15                  20

Leu Gly Ser Thr Phe Ile Val Thr Ala Gly Ala Asp Gly Ala Leu Thr
    25                  30              35                      40

Gly Thr Tyr Glu Ser Ala Val Gly Asn Ala Glu Ser Arg Tyr Val Leu
                45                  50                      55

Thr Gly Arg Tyr Asp Ser Ala Pro Ala Thr Asp Gly Ser Gly Thr Ala
            60                  65                  70

Leu Gly Trp Thr Val Ala Trp Lys Asp Asn Tyr Arg Asn Ala His Ser
        75                  80                  85

Ala Thr Thr Trp Ser Gly Gln Tyr Val Gly Gly Ala Glu Ala Arg Ile
    90                  95                  100

Asn Thr Gln Trp Leu Leu Thr Ser Gly Thr Thr Glu Ala Asn Ala Trp
105                 110                 115                 120

Lys Ser Thr Leu Val Gly His Asp Thr Phe Thr Lys Val Lys Pro Ser
            125                 130                 135

Ala Ala Ser Ile Asp Ala Ala Lys Lys Ala Gly Val Asn Asn Gly Asn
            140                 145                 150

Pro Leu Asp Ala Val Gln Gln
            155
```

(2) ANGABEN ZU SEQ ID NO: 3:

  (i) SEQUENZKENNZEICHEN:

    (A) LÄNGE: 604 Basenpaare
    (B) ART: Nucleotid
    (C) STRANGFORM: beides
    (D) TOPOLOGIE: linear

  (ix) MERKMAL:

    (A) NAME/SCHLÜSSEL: CDS
    (B) LAGE:44..499

  (ix) MERKMAL:

    (A) NAME/SCHLÜSSEL: sig_peptide
    (B) LAGE:44..115

  (ix) MERKMAL:

    (A) NAME/SCHLÜSSEL: mat_peptide

(B) LAGE:116..499

(xi) SEQUENZBESCHREIBUNG: SEQ ID NO: 3:

```
GAATTCCGCA AGGAGCACAC CCGGCTGTCC ACCTGCTGCA GAG ATG GTG CAC GCA        55
                                                   Met Val His Ala
                                                   -24

ACC TCC CCG CTG CTG CTG CTG CTG CTG CTC AGC CTG GCT CTG GTG GCT        103
Thr Ser Pro Leu Leu Leu Leu Leu Leu Leu Ser Leu Ala Leu Val Ala
-20              -15                 -10                        -5

CCC GGC CTC TCT GCC AGA AAG TGC TCG CTG ACT GGG AAA TGG ACC AAC        151
Pro Gly Leu Ser Ala Arg Lys Cys Ser Leu Thr Gly Lys Trp Thr Asn
                 1               5                   10

GAT CTG GGC TCC AAC ATG ACC ATC GGG GCT GTG AAC AGC AGA GGT GAA        199
Asp Leu Gly Ser Asn Met Thr Ile Gly Ala Val Asn Ser Arg Gly Glu
         15                  20                  25

TTC ACA GGC ACC TAC ATC ACA GCC GTA ACA GCC ACA TCA AAT GAG ATC        247
Phe Thr Gly Thr Tyr Ile Thr Ala Val Thr Ala Thr Ser Asn Glu Ile
    30                  35                  40

AAA GAG TCA CCA CTG CAT GGG ACA CAA AAC ACC ATC AAC AAG AGG ACC        295
Lys Glu Ser Pro Leu His Gly Thr Gln Asn Thr Ile Asn Lys Arg Thr
45                  50                  55                  60

CAG CCC ACC TTT GGC TTC ACC GTC AAT TGG AAG TTT TCA GAG TCC ACC        343
Gln Pro Thr Phe Gly Phe Thr Val Asn Trp Lys Phe Ser Glu Ser Thr
                65                  70                  75

ACT GTC TTC ACG GGC CAG TGC TTC ATA GAC AGG AAT GGG AAG GAG GTC        391
Thr Val Phe Thr Gly Gln Cys Phe Ile Asp Arg Asn Gly Lys Glu Val
                80                  85                  90

CTG AAG ACC ATG TGG CTG CTG CGG TCA AGT GTT AAT GAC ATT GGT GAT        439
Leu Lys Thr Met Trp Leu Leu Arg Ser Ser Val Asn Asp Ile Gly Asp
        95                  100                 105

GAC TGG AAA GCT ACC AGG GTC GGC ATC AAC ATC TTC ACT CGC CTG CGC        487
Asp Trp Lys Ala Thr Arg Val Gly Ile Asn Ile Phe Thr Arg Leu Arg
        110                 115                 120

ACA CAG AAG GAG TGAGGATGGC CCCGCAAAGC CAGCAACAAT GCCGGAGTGC            539
Thr Gln Lys Glu
        125

TGACACTGCT TGTGATATTC CTCCCAATAA AGCTTTGCCT CAGACAAAAA AAAAAAAAGG      599

AATTC                                                                  604
```

(2) ANGABEN ZU SEQ ID NO: 4:

    (i) SEQUENZKENNZEICHEN:

        (A) LÄNGE: 152 Aminosäuren
        (B) ART: Aminosäure
        (D) TOPOLOGIE: linear

    (ii) ART DES MOLEKÜLS: Protein

    (xi) SEQUENZBESCHREIBUNG: SEQ ID NO: 4:

```
Met Val His Ala Thr Ser Pro Leu Leu Leu Leu Leu Leu Leu Ser Leu
-24              -20              -15              -10

Ala Leu Val Ala Pro Gly Leu Ser Ala Arg Lys Cys Ser Leu Thr Gly
            -5                   1               5

Lys Trp Thr Asn Asp Leu Gly Ser Asn Met Thr Ile Gly Ala Val Asn
    10              15              20

Ser Arg Gly Glu Phe Thr Gly Thr Tyr Ile Thr Ala Val Thr Ala Thr
    25              30              35              40

Ser Asn Glu Ile Lys Glu Ser Pro Leu His Gly Thr Gln Asn Thr Ile
                45              50              55

Asn Lys Arg Thr Gln Pro Thr Phe Gly Phe Thr Val Asn Trp Lys Phe
            60              65              70

Ser Glu Ser Thr Thr Val Phe Thr Gly Gln Cys Phe Ile Asp Arg Asn
        75              80              85

Gly Lys Glu Val Leu Lys Thr Met Trp Leu Leu Arg Ser Ser Val Asn
    90              95              100


Asp Ile Gly Asp Asp Trp Lys Ala Thr Arg Val Gly Ile Asn Ile Phe
105              110             115             120

Thr Arg Leu Arg Thr Gln Lys Glu
            125
```

(2) ANGABEN ZU SEQ ID NO: 5:

(i) SEQUENZKENNZEICHEN:

    (A) LÄNGE: 46 Basenpaare
    (B) ART: Nucleotid
    (C) STRANGFORM: Einzelstrang
    (D) TOPOLOGIE: linear

(xi) SEQUENZBESCHREIBUNG: SEQ ID NO: 5:
CATGGCCGAA GCTGGTATCA CTGGGACCTG GTATAACCAA TGGGGG    46

(2) ANGABEN ZU SEQ ID NO: 6:

(i) SEQUENZKENNZEICHEN:

    (A) LÄNGE: 46 Basenpaare
    (B) ART: Nucleotid
    (C) STRANGFORM: Einzelstrang
    (D) TOPOLOGIE: linear

(xi) SEQUENZBESCHREIBUNG: SEQ ID NO: 6:
TCGACCCCCA TTGGTTATAC CAGGTCCCAG TGATACCAGC TTCGGC    46

(2) ANGABEN ZU SEQ ID NO: 7:

(i) SEQUENZKENNZEICHEN:

    (A) LÄNGE: 59 Basenpaare
    (B) ART: Nucleotid

(C) STRANGFORM: Einzelstrang
(D) TOPOLOGIE: linear

(xi) SEQUENZBESCHREIBUNG: SEQ ID NO: 7:
GCGCCATGGC CGAAGCTGGT ATCACTGGCA CCTGGTATAA CCAACTGGGG CGCACTTTC          59

(2) ANGABEN ZU SEQ ID NO: 8:

(i) SEQUENZKENNZEICHEN:

(A) LÄNGE: 26 Basenpaare
(B) ART: Nucleotid
(C) STRANGFORM: Einzelstrang
(D) TOPOLOGIE: linear

(xi) SEQUENZBESCHREIBUNG: SEQ ID NO: 8.
CGCGAAGCTT CAGCTGTCAT TAGCTA          26

(2) ANGABEN ZU SEQ ID NO: 9:

(i) SEQUENZKENNZEICHEN:

(A) LÄNGE: 40 Basenpaare
(B) ART: Nucleotid
(C) STRANGFORM: Einzelstrang
(D) TOPOLOGIE: linear

(xi) SEQUENZBESCHREIBUNG: SEQ ID NO: 9:
GACGGAGCTC TGACTGGGAC CTACGAACGC GCGGTTGGTA          40

(2) ANGABEN ZU SEQ ID NO: 10:

(i) SEQUENZKENNZEICHEN:

(A) LÄNGE: 40 Basenpaare
(B) ART: Nucleotid
(C) STRANGFORM: Einzelstrang
(D) TOPOLOGIE: linear.

(xi) SEQUENZBESCHREIBUNG: SEQ ID NO: 10:
GACGGAGCTC TGACTGGCAC CTACGAATGG GCGGTTGGTA          40

(2) ANGABEN ZU SEQ ID NO: 11:

(i) SEQUENZKENNZEICHEN:

(A) LÄNGE: 49 Basenpaare
(B) ART: Nucleotid
(C) STRANGFORM: Einzelstrang
(D) TOPOLOGIE: linear .

(xi) SEQUENZBESCHREIBUNG: SEQ ID NO: 11:
TTTCCATGCA TTCGCTTCGG TAGTGCCGGA TGTCCACAGC CACTGAGTG          49

(2) ANGABEN ZU SEQ ID NO: 12:

(i) SEQUENZKENNZEICHEN:

(A) LÄNGE: 40 Basenpaare
(B) ART: Nucleotid
(C) STRANGFORM: Einzelstrang
(D) TOPOLOGIE: linear

(xi) SEQUENZBESCHREIBUNG: SEQ ID NO: 12
GACGGAGCTC TGACTGGCAC CTACGAATAT GCGGTTGGTA        40

(2) ANGABEN ZU SEQ ID NO: 13:

(i) SEQUENZKENNZEICHEN:

(A) LÄNGE: 12 Basenpaare
(B) ART: Nucleotid
(C) STRANGFORM: Einzelstrang
(D) TOPOLOGIE: linear

(xi) SEQUENZBESCHREIBUNG: SEQ ID NO: 13:
GCGAAATCGA CA        12

(2) ANGABEN ZU SEQ ID NO: 14:

(i) SEQUENZKENNZEICHEN:

(A) LÄNGE: 22 Basenpaare
(B) ART: Nucleotid
(C) STRANGFORM: Einzelstrang
(D) TOPOLOGIE: linear

(xi) SEQUENZBESCHREIBUNG: SEQ ID NO: 14:
ACTAGTGTCG ATTTCGCTGC AT        22

(2) ANGABEN ZU SEQ ID NO: 15:

(i) SEQUENZKENNZEICHEN:

(A) LÄNGE: 384 Basenpaare
(B) ART: Nucleotid
(C) STRANGFORM: beides
(D) TOPOLOGIE: linear

(ix) MERKMAL:

(A) NAME/SCHLÜSSEL: CDS
(B) LAGE:1..384

(xi) SEQUENZBESCHREIBUNG: SEQ ID NO: 15:

```
ATG GCC GAA GCT GGT ATC ACT GGC ACC TGG TAT AAC CAA CTG GGG TCG        48
Met Ala Glu Ala Gly Ile Thr Gly Thr Trp Tyr Asn Gln Leu Gly Ser
    130                 135                 140

ACT TTC ATT GTG ACC GCT GGT GCT GAC GGA GCT CTG ACT GGC ACC TAC        96
Thr Phe Ile Val Thr Ala Gly Ala Asp Gly Ala Leu Thr Gly Thr Tyr
145                 150                 155                 160

GAA TCT GCG GTT GGT AAC GCA GAA TCC CGC TAC GTA CTG ACT GGC CGT       144
```

```
      Glu Ser Ala Val Gly Asn Ala Glu Ser Arg Tyr Val Leu Thr Gly Arg
                      165                 170                 175

      TAT GAC TCT GCA CCT GCC ACC GAT GGC TCT GGT ACC GCT CTG GGC TGG      192
      Tyr Asp Ser Ala Pro Ala Thr Asp Gly Ser Gly Thr Ala Leu Gly Trp
                      180                 185                 190

      ACT GTG GCT TGG AAA AAC AAC TAT CGT AAT GCG CAC AGT GCC ACT ACG      240
      Thr Val Ala Trp Lys Asn Asn Tyr Arg Asn Ala His Ser Ala Thr Thr
                      195                 200                 205

      TGG TCT GGC CAA TAC GTT GGC GGT GCT GAG GCT CGT ATC AAC ACT CAG      288
      Trp Ser Gly Gln Tyr Val Gly Gly Ala Glu Ala Arg Ile Asn Thr Gln
                      210                 215                 220

      TGG CTG TTA ACA TCC GGC ACT ACC GAA GCG AAT GCA TGG AAA TCG ACA      336
      Trp Leu Leu Thr Ser Gly Thr Thr Glu Ala Asn Ala Trp Lys Ser Thr
      225                 230                 235                 240

      CTA GTA GGT CAT GAC ACC TTT ACC AAA GTT AAG CCT TCT GCT GCT AGC      384
      Leu Val Gly His Asp Thr Phe Thr Lys Val Lys Pro Ser Ala Ala Ser
                      245                 250                 255
```

(2) ANGABEN ZU SEQ ID NO: 16:

    (i) SEQUENZKENNZEICHEN:

        (A) LÄNGE: 128 Aminosäuren
        (B) ART: Aminosäure
        (D) TOPOLOGIE: linear

    (ii) ART DES MOLEKÜLS: Protein

    (xi) SEQUENZBESCHREIBUNG: SEQ ID NO: 16:

```
      Met Ala Glu Ala Gly Ile Thr Gly Thr Trp Tyr Asn Gln Leu Gly Ser
      1                   5                   10                  15

      Thr Phe Ile Val Thr Ala Gly Ala Asp Gly Ala Leu Thr Gly Thr Tyr
                      20                  25                  30

      Glu Ser Ala Val Gly Asn Ala Glu Ser Arg Tyr Val Leu Thr Gly Arg
                      35                  40                  45

      Tyr Asp Ser Ala Pro Ala Thr Asp Gly Ser Gly Thr Ala Leu Gly Trp
                      50                  55                  60

      Thr Val Ala Trp Lys Asn Asn Tyr Arg Asn Ala His Ser Ala Thr Thr
      65                  70                  75                  80

      Trp Ser Gly Gln Tyr Val Gly Gly Ala Glu Ala Arg Ile Asn Thr Gln
                      85                  90                  95

      Trp Leu Leu Thr Ser Gly Thr Thr Glu Ala Asn Ala Trp Lys Ser Thr
                      100                 105                 110

      Leu Val Gly His Asp Thr Phe Thr Lys Val Lys Pro Ser Ala Ala Ser
                      115                 120                 125
```